# EUROPEAN PATENT SPECIFICATION

(11) **EP 3 317 402 B1**
(45) Date of publication and mention of the grant of the patent: **14.02.2024**
(21) Application number: 16741685.8
(22) Date of filing: 29.06.2016
(51) Int. Cl.: C12N 5/0784, A61K 35/15

(54) **A VIABLE CELL POPULATION, METHOD FOR PRODUCTION AND USES THEREOF**
LEBENSFÄHIGE ZELLPOPULATION, VERFAHREN ZUR HERSTELLUNG UND VERWENDUNGEN DAVON
POPULATION DE CELLULES VIABLES, PROCÉDÉ DE PRODUCTION ET UTILISATIONS DE CELLE-CI

(30) Priority: 30.06.2015 PT 2015108621
(43) Date of publication of application: 09.05.2018
(73) Proprietor: Universidade Nova de Lisboa, 1099-085 Lisbon (PT)
(72) Inventor: QUINTELA VIDEIRA, Paula Alexandra, 1150-082 Lisboa (PT); ARAUJO ALVES MARTINS DA SILVA, Mariana, 1150-082 Lisboa (PT); COSTA DE CARVALHO MARQUES, Graça Susete, 1150-082 Lisboa (PT); SANTOS FERNANDES FERRO, Tiago João, 1150-082 Lisboa (PT); CORDEIRO DA SILVA, Zélia Maria, 1150-082 Lisboa (PT); DE BARROS MONTEIRO, Mauro André, 1150-082 Lisboa (PT); FIGUEIREDO GONÇALVES, Márcia, 1150-082 Lisboa (PT); TADOKORO, Carlos Eduardo, 1150-082 Lisboa (PT); VAN KOOYK, Yvette, 1150-082 Lisboa (PT); VAN VLIET, Sandra Johanna, 1150-082 Lisboa (PT); DE JESUS MATOS, Maria Teresa, 1150-082 Lisboa (PT)
(74) Representative: Pereira da Cruz, Joao
(86) International application number: PCT/IB2016/053901
(87) International publication number: WO 2017/002045

(56) References cited:
- SANGKON OH ET AL: "Viral Neuraminidase Treatment of Dendritic Cells Enhances Antigen-Specific CD8+ T Cell Proliferation, but Does Not Account for the CD4+ T Cell Independence of the CD8+ T Cell Response during Influenza Virus Infection", VIROLOGY, vol. 286, no. 2, 1 August 2001 (2001-08-01), pages 403-411, XP055300838, AMSTERDAM, NL ISSN: 0042-6822, DOI: 10.1006/viro.2001.0992
- HÉLIO J. CRESPO ET AL: "Effect of sialic acid loss on dendritic cell maturation", IMMUNOLOGY, vol. 128, no. 1pt2, 1 September 2009 (2009-09-01), pages e621-e631, XP055300892, GB ISSN: 0019-2805, DOI: 10.1111/j.1365-2567.2009.03047.x
- CABRAL M G ET AL: "Human dendritic cells contain cell surface sialyltransferase activity", IMMUNOLOGY LETTERS, ELSEVIER BV, NL, vol. 131, no. 1, 15 June 2010 (2010-06-15) , pages 89-96, XP027076969, ISSN: 0165-2478, DOI: 10.1016/J.IMLET.2010.02.009 [retrieved on 2010-03-03]
- JENNER J ET AL: "Increased alpha2,6-sialylation of surface proteins on tolerogenic, immature dendritic cells and regulatory T cells", EXPERIMENTAL HEMATOLOGY, ELSEVIER INC, US, vol. 34, no. 9, 1 September 2006 (2006-09-01), pages 1211-1217, XP027879629, ISSN: 0301-472X [retrieved on 2006-09-01]
- DATABASE MEDLINE [Online] US NATIONAL LIBRARY OF MEDICINE (NLM), BETHESDA, MD, US; December 2010 (2010-12), STAMATOS NICHOLAS M ET AL: "LPS-induced cytokine production in human dendritic cells is regulated by sialidase activity.", XP002761584, Database accession no. NLM20826611 & JOURNAL OF LEUKOCYTE BIOLOGY DEC 2010, vol. 88, no. 6, December 2010 (2010-12), pages 1227-1239, ISSN: 1938-3673
- MARIANA SILVA ET AL: "Sialic acid removal from dendritic cells improves antigen cross-presentation and boosts anti-tumor immune responses", ONCOTARGET, vol. 7, 17 May 2016 (2016-05-17), page 27, XP055300997, DOI: 10.18632/oncotarget.9419

## Description

### Technical Field

The present disclosure relates to a method for production of a viable cell population, suitable for cellular vaccine development. The viable cell population obtained by the method of the present disclosure has low sialic acid content, is loaded with specific antigens, shows higher antigen presentation, high maturation and co-stimulatory properties, and less tolerogenic properties.

The viable cell population directly obtained by the method of the present claims.

### Background

Dendritic cells (DC) are a population of hematopoietic derived white blood cells that are the most potent professional antigen presenting cells.

DC are capable of presenting antigens to both helper (CD4⁺) and cytotoxic (CD8⁺) T cells via Major Histocompatibility Complex (MHC) class II or class I, respectively (Steinman & Cohn, 1973; Steinman & Cohn, 1974; Steinman *et al.,* 1974).

DC constitute a heterogeneous blood cell population, comprising the CD11c⁺ myeloid DC, the CD141⁺ myeloid DC and the CD303⁺ plasmacytoid DC. However, blood DC are a rare population, thus methods have been developed to differentiate blood precursor cells, such as monocytes, into DC (Sallusto & Lanzavecchia, 1994).

Presently it is well accepted that blood monocytes cultivated with granulocyte macrophage colony-stimulating factor (GM-CSF) and interleukin (IL)-4 differentiate into immature DC *in vitro* (Sallusto & Lanzavecchia, 1994). With this protocol, it became possible to obtain an elevated number of cells, increasing the knowledge on means to modulate DC function and to boost the development of therapeutic applications based on DC.

The understanding that DC have the ability to elicit anti-tumour adaptive immune responses, together with the development of specific protocols for the production of these cells, has brought the idea of their application in cancer immunotherapy (Banchereau & Steinman, 1998; Steinman, 1996) and at the present time, a considerable number of studies investigate the potential of DC as cancer vaccines (Steinman & Banchereau, 2007).

The first DC-based vaccine against cancer receiving marketing approval, was Sipuleucel-T (Provenge), developed by Dendreon Corp. (Approval Letter by Food and Drug Administration. 2010-04-29). Sipuleucel-T consists in using DC from patients (autologous); obtained by differentiation of monocyte precursors *in vitro* and *ex vivo;* stimulated with a fusion protein consisting of GM-CSF and the tumour antigen, prostatic acid phosphatase (PAP). Sipuleucel-T has shown to be effective in patients with metastatic prostate cancer, by extending the median survival by 4.1 months and with an extended survival up to 3 years in some cases (Jahnisch *et al*., 2010). Besides Sipuleucel-T, a number of other companies are developing DC-based vaccines using similar approaches, such as Geron Company with GRNVAC1 and GRNVAC2 vaccines, consisting of DC expressing the telomerase gene.

These DC-based vaccines have proven to be safe and to induce anti-tumour immunological responses. However, despite being a promising technology, experts in the field refer that the methods used to obtain DC-based vaccines are relatively uneasy, since they have shown limited efficacy in patients and usually require an expensive formulation. One of the reasons for this limited efficacy is the inefficient maturation of DC used in vaccination protocols.

DCs if not properly matured induce a tolerogenic immune response, which abrogates cytolitic activity of T cells against tumour cells. In this context, inefficient maturation may be related with aspects such as: i) insufficiency or inefficiency of the maturation stimuli used *in vitro* considering that, as mentioned below, at present there is no satisfying method capable of stimulating all aspects of DC maturation or ii) DC refraction to maturation stimuli, as it is the case of DCs from cancer patients due to the immunosuppressive environment induced by the tumour cells.

To efficiently induce anti-tumour responses, DC need to undergo phenotypic and physiological changes, characterized by the up-regulation of antigen presenting MHC class I and class II molecules to present antigens (signal 1) to T cells, and co-stimulatory molecules (signal 2), such as CD86 and CD40, which promote DC interaction with T cells (Acuto *et al.,* 2003), as well as an increased ability to secrete cytokines (signal 3).

For the purpose of inducing anti-tumour immune responses, the expression of pro-inflammatory cytokines by DC, such as IL-12, is preferably higher than anti-inflammatory cytokines such as IL-10. IL-12 is actually required for *in vivo* antitumor activity by inducing human cytotoxic T cell activation, while IL-10 renders DCs with a tolerogenic profile.

Besides the cytokines expressed by DC, the profile of cytokines expressed by helper T lymphocytes are also important to assist anti-tumour immune responses. This profile can be broadly subdivided into Th1-type cytokines and Th2-type cytokines. Th1-type cytokines tend to produce pro-inflammatory responses responsible for killing tumour cells. Interferon gamma (IFN-y) is one of the major examples of Th1 cytokines.

While the previously referred protocol used to differentiate monocytes into DC is already well established, there is no satisfying method to mature DC that is capable of simultaneously stimulate all aspects of DC maturation (deploying signals 1, 2 and 3 simultaneously).

Several strategies have been engineered to obtain higher amounts and better matured DC, mainly aiming to develop efficient autologous anti-tumour immunotherapy.

Jonuleit H. *et al.* have described a maturation protocol using a composition comprising a cytokine cocktail with tumour necrosis factor (TNF)-α, IL-1 β, IL-6 and prostaglandin (PG) E2. This composition promotes the *in vitro* maturation of DC, increasing the expression of MHC class II and co-stimulatory markers (Jonuleit *et al.,* 1997).

Document US20140302096A1 describes an alternative method using a composition comprising TNF-α, IL-1β, PGE2, IFN-y, and TLR7/8 agonists, which increases the expression of MHC class II, co-stimulatory markers and IL-12.

Document US20140220675 A1 proposes a method to produce a population of DC capable of secreting high amounts of IL-12 and inducing the activation of cytotoxic T cells by using a combination of maturing agents comprising an array of type I interferons.

Another related strategy (document WO2003022215 A3) refers to a specific combination of maturation factors, including the use of cytokines simultaneously with Bacillus Calmette-Guérin (BCG), which would theoretically activate intracellular DC's signaling pathways that are mediated by TLRs (Toll-like receptors).

Glycosylation of cell-membrane proteins constitutes the most common posttranslational modification and plays a key role in several immune processes. Specifically, DC show a specific glycan profile at cell surface that can be modulated during development, maturation and immune regulation (Varki & Gagneux, 2012). Typically, the terminal positions of eukaryotic cell surface glycoconjugates are occupied by a negatively charged and non-reducing monosaccharide named sialic acid (Varki *et al.,* 2009).

"Sialic acid" refers to a large family of monosaccharides (or sugars), with nine carbons, derivatives of neuraminic acid, being the N-acetylneuraminic acid the most common in humans. Sialic acid plays an important role in host/pathogen interaction and recognition and is a fundamental determinant for a number of immune receptors with known involvement in cellular adhesiveness and cell-to-cell interactions, such as Selectin and Siglec receptors. Sialic acid recognition occurs either on glycoconjugates of the same cell (*in cis*) or other cells (*in trans*)*.* Sialic acid content depends on the expression of a set of different sialyltransferases (enzymes that transfer sialic acids) localized at Golgi or/and sialidases (enzymes that cleave sialic acid linkages) located in the cytoplasm, membrane or lysosomes. Besides this intrinsic regulation, sialic acid content is extrinsically modified by external factors, such as soluble sialyltransferases present in serum and soluble sialidases expressed by the host or pathogens. The term desialylation used in this work refers to the enzymatic removal of terminal sialic acid residues from glycoproteins or glycolipids, on a reaction catalyzed by extrinsic sialidase enzymes.

Sialic acids are involved in many cellular functions, both in physiological and pathological processes, including the regulation of the immune system and triggering progression of certain infections and diseases (Varki, 2008; Varki & Varki, 2007). These terminal sugars can mask underlying structures, thus preventing cell-to-cell interactions through non-specific repulsive effects or by preventing recognition by other molecules such as lectins (Dall'Olio & Chiricolo, 2001; Lehmann *et al.,* 2006; Schauer, 2009).

Videira P.A. *et al.* demonstrated that the surfaces of human monocyte-derived DC are highly sialylated and that removal of these sialic acids by sialidase resulted in induction of DC maturation. They also reported that the resulting induction of maturation decreased the capacity of DCs in 25% to uptake non-self-protein antigens, such as the ovalbumin protein, the polysaccharide dextran and the water soluble dye, Lucifer Yellow, which are internalized by specific endocytic routes (Videira *et al.,* 2008).

Crespo H. *et al.* described that the inactivation of the sialyltransferases ST3Gal.1 and ST6Gal.1 caused a more mature phenotype in murine DC (Crespo *et al.,* 2009). Cabral *et al.* demonstrated that desialylation strongly stimulates human monocyte derived-DC maturation and the production of several cytokines, including IL-10, via the translocation of NF-κB transcription factor to the nucleus (Cabral *et al.,* 2013).

Other documents report the enzymatic treatment of other cell types with glycan modifying enzymes. Document US 8084236 B2 describes compositions and methods for modifying cell surface glycans, applicable to improve cell migration, and with no impact in neither cell maturation nor immune modulation.

The use of sialidase was only proposed in dairy technology (document WO 2011095477 A1). By contrast the use of sialidase inhibitors, which abrogates the activity of sialidase on cells surface glycans is described in some documents for viral infection therapy (document WO 2011081083 A1) and to improve platelet storage (document CN 103702556 A).

Document SANGKON OH ET AL: "Viral Neuraminidase Treatment of Dendritic Cells Enhances Antigen-Specific CD8+ T Cell Proliferation, but Does Not Account for the CD4+ T Cell Independence of the CD8+ T Cell Response during Influenza Virus Infection", VIROLOGY, vol. 286, no. 2, 1 August 2001 discloses viral neuraminidase (a sialidase) treated dendritic cells.

Document JENNER J ET AL: "Increased alpha2,6-sialylation of surface proteins on tolerogenic, immature dendritic cells and regulatory T cells", EXPERIMENTAL HEMATOLOGY, ELSEVIER INC, US, vol. 34, no. 9, 1 September 2006 discloses mature and immature monocyte-derived dendritic cells, wherein specifically the mature dendritic cells were having a down regulated expression of α-2,6 sialic acid.

Document DATABASE MEDLINE [Online] US NATIONAL LIBRARY OF MEDICINE (NLM), BETHESDA, MD, US; December 2010 (2010-12), STAMATOS NICHOLAS M ET AL: "LPS-induced cytokine production in human dendritic cells is regulated by sialidase activity." XP002761584, discloses that sialidase mediated change in sialic acid content of specific cell surface glycoconjugates in DCs regulates LPS-induced cytokine production, thereby contributing to development of adaptive immune responses.

Dendritic cells (DC) have the potential to mobilize different arms of the immune response against tumour cells, and are therefore used as cell vaccines. However, at the clinical setting, DC-based vaccines are limited in inducing effective responses, due to their low capacity to present antigens, low co-stimulation and low cytokine expression.

In order to solve the above-mentioned shortcomings and to provide DC with full capacity to elicit effective responses, the present disclosure provides a method for stimulating all aspects of DC maturation: 1) higher antigen presentation through MHC class I and class II, 2) increased expression of co-stimulatory molecules and 3) the secretion of Th1 cytokines.

These facts are disclosed in order to illustrate the technical problem addressed by the present disclosure.

### General description

The three strategies mentioned above explore different ways of producing high quality mature DC. However, the modifications of exogenous glycans in DC, and in particular their sialic acid-containing sugars, has never been explored to produce DC-based vaccines.

The present disclosure describes a protocol using an enzymatic (sialidase) treatment which unexpectedly improves the antigen presentation through MHC class I and class II, including the cross presentation of antigens. Higher antigen presentation, through either MHC maximizes the efficiency of DCs as antigen presenting cells, thus reducing the dosage required for vaccination. The disclosure also improves the expression of co-stimulatory molecules, important for efficient stimulation of T cells. Furthermore, with the present disclosure there is an increased expression of pro-inflammatory cytokines and an unexpected decrease in the expression of anti-inflammatory cytokines, such as IL-10, benefiting anti-tumour immune responses. Thus this disclosure is better than the ones described above because it improves simultaneously the expression of signal 1, 2 and 3.

The present disclosure can be applied before or even after phagocytosis of antigen, in contrast to other inventions that can only be applied after antigen upload. This is an advantage that allows maturing DC prior to the antigen upload, thus minimizing the problem of immune suppression posed by tumour antigens that, when given to DC abrogate its maturation (i.e. are tolerogenic).

Unexpectedly, in the present disclosure the sialidase treatment does not affect the uptake of tumour antigens, which may be due to the fact that tumour antigens are internalized by several endocytic routes, due to their complex composition. The fact that sialidase treatment does not affect phagocytosis of tumour antigens, presents an advantage compared to other maturation strategies that abrogate antigen internalization and thus have to be performed after upload of antigens. This disclosure thus allows maturing DC prior to the antigen upload, thus minimizing the problem of immune suppression, posed by tolerogenic tumour antigens.

Unexpectedly, in the present disclosure the production of IL-10 was reduced in 50% in DC treated with sialidase and uploaded with tumour antigens, compared to untouched DC, suggesting that other signaling pathways are also activated. Decreased IL-10 expression is an advantage since IL-10 is a Th2 cytokine that contributes to immune tolerance. On the other hand, IL-10 stimulates tumour cell proliferation and inhibits cell apoptosis.

Sialidase treatment of DC, followed by uptake of tumour antigens as a composition to stimulate all aspects of DC maturation, i.e., signal 1, 2 and 3, including lower levels of IL-10 and cross-presentation of antigens and the effect of this composition to boost T cell responses against tumour cells has not yet been documented. The present disclosure describes composition to stimulate all aspects of DC maturation, i.e., signal 1, 2 and 3, including the effect of this composition to boost T cell responses against tumour cells, which has not yet been documented.

The present disclosure relates to a method for production of viable cell population wherein the population of viable cells is obtainable by the maturation of the dendritic cells with sialidase during 45 minutes to overnight, in particular 45 minutes to 16 hours at 37 °C and wherein the sialidase concentration vary between 0.002 U/mL - 0.005 U/mL per 1×10⁶ of dendritic cells or T2 cells.

The viable cell population obtained by the method disclosed in the present application have surprisingly the following features:
the expression of MHC class II molecules is up to 1.7 or 1.8 fold higher,
the stability of MHC class I on cell surface, in particular on the DC cell surface, is up to 52% higher, preferably up to 100% higher, more preferably 146% higher,
the expression of co-stimulatory molecules is 1.3 to 1.5 fold increased, in particular the expression of the co-stimulatory molecules CD80 and CD86 is increased 1.2 and 1.1-fold, respectively;
the secretion of pro-inflammatory cytokines is up to 1.8fold increased, in particular is increased up to 2.5 fold, more particularly the secretion of the pro-inflammatory cytokine IL-12 is increased up to 2.5-fold;
the secretion of anti-inflammatory cytokines is 1.4 to 1.9 fold decreased, in particular the secretion of anti-inflammatory cytokine IL-10 is 1.4- fold decreased; and
the ability for antigen cross-presentation is up to 113% higher.

Furthermore, the population obtained by the method of the present disclosure surprisingly also shows:
the ability for antigen cross-presentation is increased up to 1.7-fold,
the ability to boost antigen specific CD4+ T cell proliferation is increased 1.5-fold,
the ability to boost the secretion of IFN-γ and TNF-α by antigen specific T cell is increased 1.3- and 2.1-fold, respectively, and
the ability to induce T cell cytotoxicity against tumour cell is improved 1.2-fold.

The viable cell population obtained by the method of the disclosure may comprise up to 50% of sialic acids on a cell surface of a cell treated with sialidase/sialic acids on a cell surface of a cell non-treated with sialidase, preferably 40% of sialic acids on a cell surface of a cell treated with sialidase/sialic acids on a cell surface of a cell non-treated with sialidase, more preferably up to 30% of sialic acids on a cell surface of a cell treated with sialidase/sialic acids on a cell surface of a cell non-treated with sialidase, even more preferably up to 20% of sialic acids on a cell surface of a cell treated with sialidase/sialic acids on a cell surface of a cell non-treated with sialidase.

In an embodiment, the removing step of α2,3-sialic acid, α2,6-sialic acid and α2,8-sialic acid from a cell surface precedes the maturing step.

In an embodiment, this comprises a loading step of an antigen at the surface of the population of viable dendritic cells.

In an embodiment, the loading step of the antigen is performed at 37°C, 30 minutes up to 4 hours, and up to 100 µM of said antigen.

The dendritic cell population directly obtained by the method of the claimsis isolated from blood, from differentiation of monocytes, from CD34⁺ hematopoietic stem cells, or from the peripheral blood.

The monocytes may be isolated from peripheral blood or from cord blood.

The CD34⁺ hematopoietic stem cells may be isolated from cord blood or from peripheral blood.

In an embodiment, the maturing step is performed for 45-80 minutes a 37°C, preferably at 45-60 minutes at 37°C.

In an embodiment, the sialidase enzyme may be a recombinant or purified from a biological source, in particular wherein the biological source of sialidase may be from bacterial, viral or mammalian, including human sources. Sialidase cleaves cell surface sialic acids. The cell surface sialic acid residues that are removed comprise α2,3- and α2,6-linked sialic acids and in a lesser extent α2,8- linked sialic acids.

### Description of the drawings

Without intent to limit the disclosure herein, this disclosure presents attached drawings of illustrated embodiments for an easier understanding. The examples relating to T2 cells are presented for illustration purpose only.

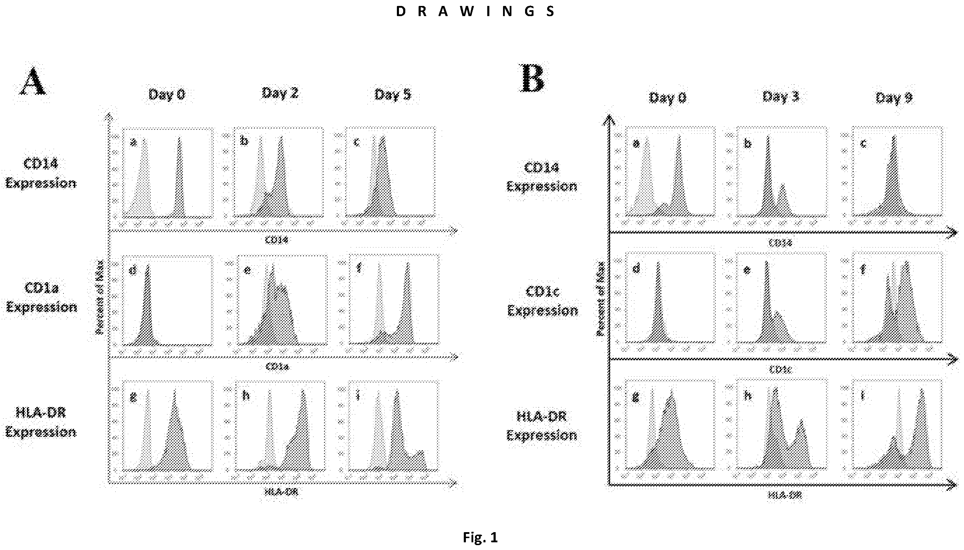

**Figure 1****.** Human DCs are obtained by differentiation of blood precursors. The blood precursors were monocytes or CD34⁺ cells. Monocytes were isolated from (A) adult peripheral blood cells or from (B) cord blood cells using magnetic beads conjugated to antibodies against CD14. Monocytes were cultured at a concentration of 1 × 10⁶ cells/ml in media supplemented with 750 U/mL of IL-4 and 1000 U/mL of GM-CSF, at 37ºC, in a humidified atmosphere with 5% CO₂. Cells were analyzed at different time points throughout the differentiation period by flow cytometry, using antibodies against: CD14, a marker mainly expressed by monocytes; CD1a or CD1c, markers expressed by human DC and HLA-DR (MHC class II) an antigen presenting molecule. During differentiation into DC, monocytes lose their expression of CD14 and acquire significant expression of CD1a or CD1c, also increasing the expression of HLA-DR. Adult monocytes after five days in culture, acquire a DC phenotype, while monocytes isolated from cord blood acquire a DC phenotype after seven days. The differences in time for differentiation between monocytes from adult blood and cord blood are due to the cell immaturity. The differentiation yield is above 90%, meaning that almost every monocyte differentiates into DC. The figure shows representative histograms of at least three independent assays. Positive staining is represented in dark grey and isotype control is shown in light grey.

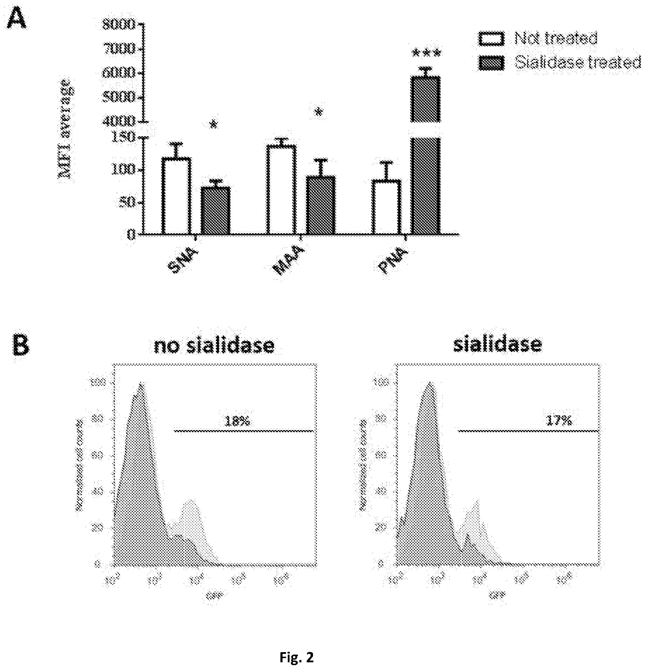

**Figure 2****.** Sialidase treatment efficiently removes α2,6- and α2,3-linked sialic acids from the DC cell surface and does not affect endocytosis capacity. (A) Flow cytometric analysis of DC obtained from differentiation of human monocytes (MoDC), stained with fluorescent Sambucus *nigra lectin* (SNA; recognizing α2,6-sialic acids), *Maackia amurensis lectin* (MAA; recognizing α2,3-sialic acids) and *Peanut agglutinin lectin* (PNA; recognizing unsialylated core 1 structures). The degree of desialylation is quantified based on the ratio between the mean fluorescence intensity (MFI) ± SEM of DC after sialidase treatment compared to untreated DC. Data shows that sialidase treatment leads to a 50 ± 26 % and a 45 ± 10 % decrease in SNA and MAA staining, respectively, while PNA staining increases 70 ± 13 fold. Statistical significance was determined using a two-tailed paired t-test (*P < 0.05 or ***P < 0.0001) and refers to the difference between untreated and sialidase treated MoDC (n≥3) (B) Sialidase treatment does not affect the capacity of DC to endocytose tumour cell lysates. DC previously treated or not with sialidase were incubated with fluorescent tumour cell lysates, for 3 hours at 37ºC (condition that favours endocytosis) and 4ºC (condition where endocytosis is inhibited), and analysed by flow cytometry. The capacity for endocytosis was quantified based on the number of DC which showed significant increase in the MFI, after endocytosis experiments at 37ºC. Increased MFI means that cells have internalized fluorescent antigens. The values obtained in assays conducted at 4ºC, which could evaluate the occurrence of eventual unspecific binding to cell surface, showed negligible fluorescence. Data showed that there are no significant differences between assays conducted with DC not treated (left graph) or treated with sialidase (right graph).

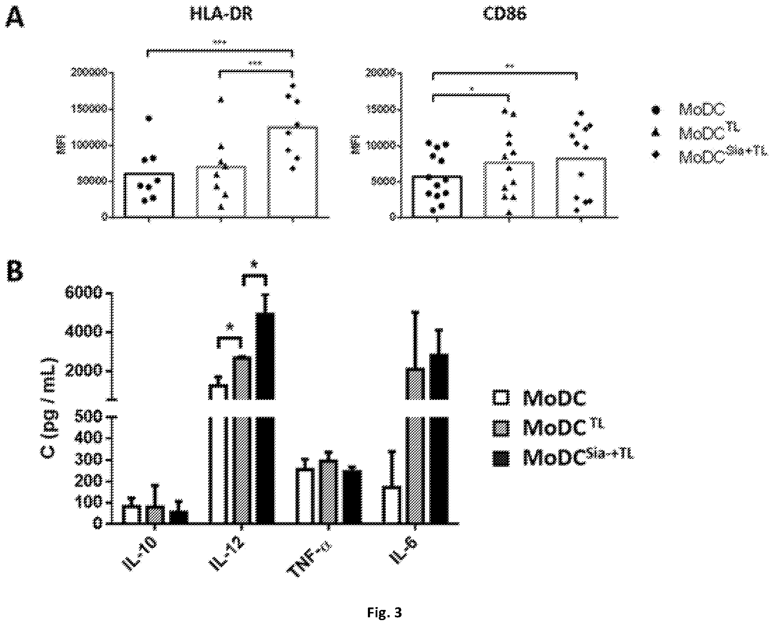

**Figure 3****.** Desialylated human MoDC loaded with whole tumour cell antigens express a more mature profile and have higher secretion of pro-inflammatory cytokines. MoDC were first treated with sialidase (Sia), for 1 hour at 37ºC or left untreated, followed by loading with lysates of the tumour cell line MCF-7 (TL), as a source of whole tumour cell antigens, as described in Example 5. Control experiments with non-sialidase treated and unloaded MoDC were run in parallel (represented in the figure as MoDC). (A) Flow cytometric analysis of the expression of antigen presenting molecule HLA-DR (MHC class II), and co-stimulatory molecule CD86. Untreated and unloaded MoDC have the lowest HLA-DR and CD86 expression, namely 61082 ± 13341 and 5749 ± 912.4 of mean fluorescence intensity (MFI), respectively. By loading MoDC with tumour cell lysates (MoDC^{TL}), it was achieved a MFI of 70432 ± 16335 and 7652 ± 1341, respectively, which represents an increase of 1.2-fold for the expression of HLA-DR and 1.3-fold for the expression of CD86. The highest expression of HLA-DR and CD86 was achieved with sialidase treated MoDC loaded with tumour antigens (MoDC^{Sia+TL}), resulting in a MFI of 125049 ± 14991 for HLA-DR and of 8208 ± 1447 for CD86 expression, which represents 2-fold and 1.4-fold increase compared to untreated and unloaded MoDC. Thus comparing MoDC^{Sia+TL} versus MoDC^{TL}, it can be observed that the sialidase treatment improved the expression of HLA-DR in 1.8-fold and of CD86 in 1.1-fold. Graph values represent the MFI ± SEM of at least five independent assays. Statistically significant differences were calculated using a two-tailed paired t-test (***P < 0.0001). (B) MoDC secretion of IL-10, IL-12, TNF-α and IL-6. Cytokine secretion was evaluated by ELISA technique in culture supernatants obtained 24 hours after the treatments. The expression of IL-12 was 2674.00 ± 46.25 in MoDC loaded with tumour antigens (MoDC^{TL}) and 4914.83 ± 1039.63 pg/mL in MoDC treated with sialidase and loaded with tumour antigens (MoDC^{Sia+TL}). Thus, the sialidase treatment increased the expression of IL-12 1.8 fold. The expression of IL-10 was 79.50 ± 44.02 pg/mL in MoDC^{TL} and 56.44 ± 49.44 pg/mL in MoDC^{Sia+TL}. Thus, the sialidase treatment decreased 1.4-fold the IL-10 secretion. The IL-6 and TNF-α expression was not significantly altered after sialidase treatment, showing that there is not excessive pro-inflammatory cytokines release after sialidase treatment. Graph values represent the mean concentration (pg/mL) ± SEM of at least three independent assays. Statistically significant differences were calculated using a two-tailed paired t-test (*P < 0.05).

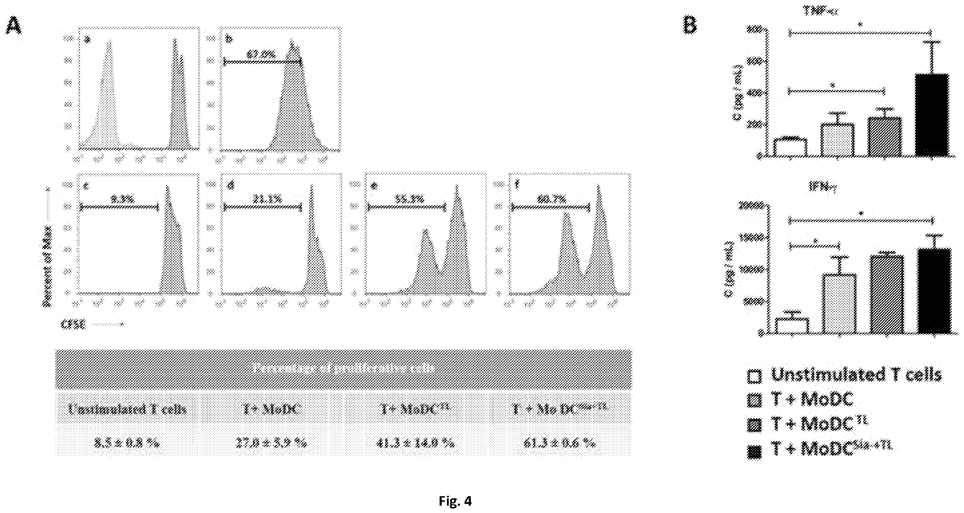

**Figure 4****.** Sialidase treatment of human MoDC loaded with whole tumour cell antigens induces T cell activation. MoDC were first treated with sialidase (Sia), for 1 hour at 37ºC or left untreated, followed by loading with lysates of the tumour cell line MCF-7 (TL), as source of whole tumour cell antigens, as described in Example 5. MoDC were then used in co-cultures with autologous T cells for 4-8 days in the presence of low-dose IL-2. (A) Induction of T cell proliferation by MoDC. T cells were previously labeled with CFSE and the progeny, i.e., the percentage of T cells that proliferated was estimated by flow cytometry, based on the dilution of CFSE. The histograms show representative experiments with T cells that were co-cultured with: unloaded MoDC (panel d), with MoDC loaded with tumour cell line MCF-7 lysates, as source of whole cell antigen (panel e) or with sialidase treated MoDC loaded with MCF-7 cell line lysates (panel f). Unstimulated T cells (panel c) served as a negative control and phytohaemagglutinin-stimulated T cells (panel b) as a positive control. In panel a, it is represented the parental population (dark grey) and the unstained control (light grey). Using MoDC loaded with tumour cell lysates (MoDC^{TL}) resulted in 41.3 ± 14 % of proliferative T cells, a 4.9-fold increase when compared to T cells in culture alone (9.5 ± 0.8 %), and a 1.5-fold increase when compared to T cell in culture with MoDC (27.0 ± 5.9 %). Moreover, treating MoDC with sialidase and loading them with tumour cell lysates (MoDC^{Sia+TL}) led to a percentage of proliferative T cells of 61.3 ± 0.6 %, an increase of 7.2-fold when compared to T cells alone, 2.3-fold, when compared to T cells in culture with MoDC and 1.5-fold compared to T cells in culture with MoDC^{TL}. Tabled values represent the mean percentage of proliferative cells ± SEM of 2 independent assays. (B) Th1 cytokines secreted in DC: T cell co-culture. The cytokines secreted into the co-culture supernatants, following T cell stimulation with MoDC were measured by ELISA. T cell boosted by MoDC expressed 200.1 ± 162.0 pg/mL of TNF-α, which is 1.9-fold more than T cell alone. When boosted by MoDC^{TL}, or MoDC^{Sia+TL} the TNF-α secretion increases to 241.9 ± 139.4 and 514.6 ± 246.0 pg/mL, respectively. Thus sialidase treatment, prior to the upload of DC with tumour antigen improves 2.5-fold the ability of autologous T cells to secrete TNF-α. In the case of IFN-y, T cells boosted by Mo-DC secreted 7643.6 ± 3019.6 pg/mL, which is 3.4-fold more than T cells alone. When boosted by MoDC^{TL}, or MoDC^{Sia+TL} the secretion of IFN-γ increases to 9620.1 ± 584.1 or 13166.6 ± 1866.7 pg/mL, respectively. Thus sialidase treatment prior to the upload of DC with tumour antigen improves 1.6-fold their capacity to activate the secretion of IFN-γ by autologous T cells. Graph values represent the mean concentration (pg/mL) ± SEM of six independent experiments. Statistically significant differences were calculated using a two-tailed paired t-test (*P < 0.05).

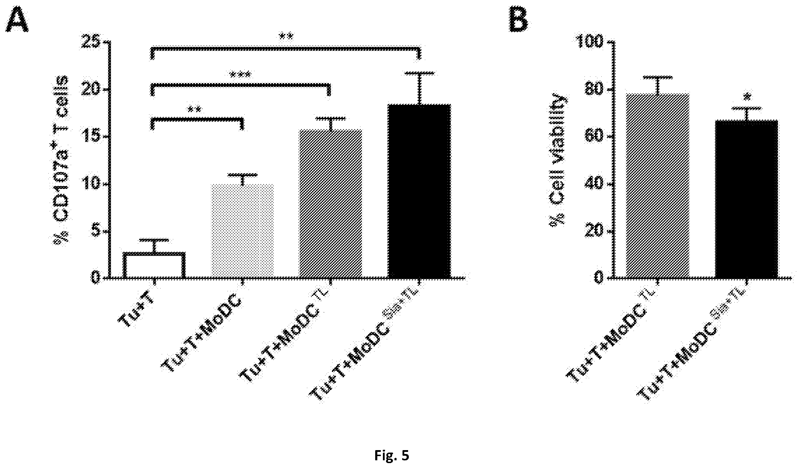

**Figure 5****.** Sialidase treatment of human MoDC loaded with whole tumour cell antigens improves T cell cytotoxicity against tumour cells. MoDC obtained from HLA-A*02:01 donors were treated with sialidase (Sia), for 1 hour at 37ºC or left untreated, followed by loading with lysates of the breast cancer cell line MCF-7 (TL), as a source of whole tumour cell antigens, as described in Example 5. (A) T cells boosted with desialylated MoDC show higher cytotoxicity against tumour cells. Autologous CD3 T cells were co-cultured with untreated MoDC, with MoDC loaded with tumour cell line MCF-7 lysates, or MoDC that were sialidase treated and loaded with tumour cell line MCF-7 lysates. After 3 weeks, CD3⁺ T cells were co-cultured with MCF-7 cells in a ratio of 1 tumour cells: 10 T cells; non-stimulated T cells were cultured with MCF-7 as control. Cells were co-cultured for 5 hour, after which, the degranulation of cytotoxic T cells against tumour cells was determined by the percentage of cells expressing the CD107a marker. The fact of co-culturing tumour cells with unstimulated T cells, led to the degranulation of 2.62 ± 1.49 % of the T cells. However, when using T cells that had been previously stimulated with MoDC the percentage of cytotoxic T cells that had degranulated, when in contact with tumour cells increased to 9.84 ± 1.15 % (3.8-fold increase); when using T cells stimulated with MoDC^{TL}, it increased to 15.55 ± 1.4 %, (5.9-fold increase), and the highest increase (7-fold increase) was achieved when using T cells stimulated with MoDC^{Sia+TL}, leading to the degranulation of 18.29 ± 3.46 % of the T cells. Thus T cells boosted with DC that were sialidase treated prior to the upload with tumour antigens, show 18% better ability to kill tumour cells than those that were boosted with DC loaded with antigens only. Graph values represent the percentage ± SEM of CD107a⁺ T cells of at least four independent assays. Statistically significant differences were calculated using a two-tailed paired t-test (**P < 0.001 and ***P < 0.0001). (B) Survival of tumour cells co-cultured with T cells. By measuring the expression of the green fluorescent protein (GFP), in GFP-expressing tumour cells, it is possible to infer which cells are untouched, i.e. survive or started apoptosis. GFP is a dye whose fluorescence is pH sensitive, decreasing significantly when the pH of the cell cytoplasm decreases, as it is the case of T cell-dependent apoptosis. By evaluating the intensity of the GFP reporter in tumour cells, it was possible to determine which tumour cells survived or were not entering into apoptosis. Hence, tumour cells in co-culture alone have a percentage of survival superior than 94%. However, upon co-culture with T cells previously stimulated with MoDC^{TL}, the survival of the tumour cell decreases to 77.53 ± 7.77 %, and it still further decreases to 66.50 ± 5.73 % when T cells had been stimulated with MoDC^{Sia+TL}. In agreement with previous results, it is possible to infer that T cells boosted with MoDC that were sialidase treated previously to the upload with tumour antigen, show 17% better ability to reduce tumour cell survival than those that were boosted with MoDC only loaded with tumour antigens. Graph values represent the percentage ± SEM of viable tumour cells (GFP⁺) of four independent assays. Statistically significant differences were calculated using a one-tailed paired t-test (*P < 0.05).

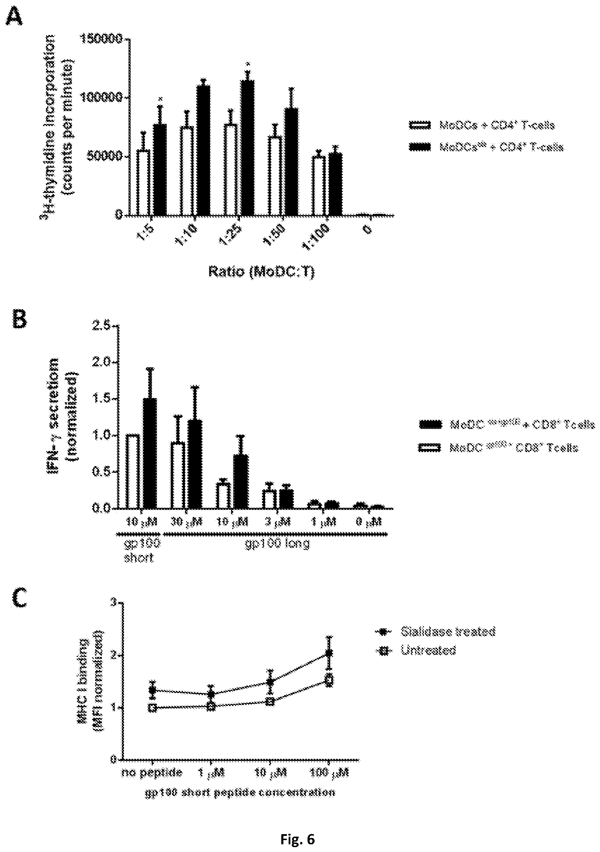

**Figure 6****.** Sialidase treatment of MoDC improves the ability to activate tumour antigen gp100-specific T cells. (A) Improved proliferation of autologous helper CD4⁺ T cells, challenged for 3 days, with sialidase treated MoDC. The proliferation of CD4⁺ T cells, challenged with different ratios of MoDC treated (MoDC^{sia}) or untreated with sialidase (MoDC), was measured, by using the [³H]thymidine uptake assay (When comparing different MoDC:T cell ratios, the treatment of MoDC with sialidase results in an increase of the scintillation counts per minute, relatively to the untreated condition. The lowest increase was 5% and occurred in the 1:100 ratio (49838.8 ± 5237.4 scintillations counts per minute using untreated MoDC, vs. 52273.2 ± 6622.5 in the sialidase treated MoDC). With the 1:50 ratio there was an increase of 36% (66786.0 ± 10693.6 vs. 90686.3 ± 17303.8 scintillations counts per minute (untreated vs. sialidase treated MoDC)), the 1:5 ratio resulted in a 40% increase (55014.4 ± 15725.9 vs. 77147.2 ± 15801.5 scintillations counts per minute (untreated vs. sialidase treated MoDc)) and with the 1:10 ratio there was a 46% increase (75085.2 ± 13916.8 vs. 109758.4 ± 5777.6 scintillations counts per minute (untreated vs. sialidase treated MoDC)). Finally, the highest increase of the number of scintillations per minute occurred with the 1:25 ratio and corresponded to 48% (77250.3 ± 12477.3 *vs.* 114241.7 ± 8193.4 scintillations counts per minute (untreated vs. treated)) Graph values represent scintillation counts per minute ± SEM of three independent assays. The statistical significance, determined with two-tailed paired t-test (*P < 0.05), refers to the difference between untreated and sialidase treated MoDCs. (B) Improved antigen cross presentation by sialidase treated MoDCs. Gp100-specific CD8⁺ T cell clones were co-cultured overnight with gp100-loaded MoDC, that have been previously treated or not with sialidase (MoDC^{sia+gp100} and MoDC^{gp100}, respectively). Different concentrations of long gp100 peptide, which is cross-presented to CD8⁺ T cells, were used. Short gp100 peptide was used as a control for the functionality and antigen specificity of CD8⁺ T cell clones. The secretion of IFN-γ cytokine was measured by ELISA, as a measure to evaluate the activation of CD8⁺ T cell clones. Results were normalized to the IFN-y levels of the incubation of T cells with MoDC loaded with the short peptide, without sialidase treatment and refer to the secretion ± SEM of INF-y for three independent experiments. With the short peptide (positive control), using sialidase treated MoDC, the expression of IFN-Y increased 49%, relatively to the condition with untreated MoDC. When no peptide was used (0µM;negative control), the secretion of IFN-y was almost inexistent, dropping to 4% and 2% (96% and 98% decrease), for untreated and sialidase treated MoDC, respectively. Regarding the remaining conditions, testing different long peptide concentrations, the results show that there was an increase of IFN-Y production with higher concentration of the long peptide. Taking the IFN-y levels obtained using MoDC incubated with the short peptide as a reference (100%), we observed that with the highest concentration tested for the long peptide (30µM), there were a 10% decreased when the untreated MoDC were used, and a 19% increase for the sialidase treated MoDC condition. With a long peptide concentration of 10 µM, there was also a decrease when comparing to the control, corresponding to reductions of 66% and 28% for the untreated and sialidase treated conditions, respectively. Using a concentration of 3µM of the long peptide, it decreased the IFN-Y production 76% and 75% (in conditions with untreated and sialidase treated MoDC, respectively). With a 1µM concentration of the long peptide, there were 94% and 93% decreases relatively to the control condition with the short peptide. For every concentration where the long peptide was tested, the sialidase treatment of the MoDC resulted in an increase of IFN-Y secretion. Thus, by ascending order of concentration (1µM, 3µM, 10µM and 30µM), the increases are of 12%, 2%, 113% and 33%, respectively in the sialidase treated compared to the untreated MoDCs. Therefore the production of IFN-y is improved up to 113% by cytotoxic CD8⁺ T cell clones stimulated with sialidase treated MoDCs. (C) gp100 peptide bound MHC-I is more stable on sialidase treated T2 cells. T2 binding assays were performed by incubating cells, treated or not with sialidase, with different concentrations of gp100 short peptide in the presence of β-microglobulin for 3 to 4 hours at 37 °C. After incubation, T2 cells were washed and stained with anti-HLA-A*02 antibody, and analysed by flow cytometry. Expression of HLA at cell surface was used a measure to evaluate MHC stabilization. Data were normalized to the condition without peptide (no peptide) and without sialidase treatment. A higher stabilization of the HLA-A*01 molecule was observed after sialidase treatment, which ranged between 23% (1 µM of peptide) to 52% (100 µM of peptide). Using an intermediate peptide concentration (10 uM), the sialidase effect resulted in a stabilization effect of 38%. Graphs show the mean fluorescence intensity ± SEM of 4 independent experiments.

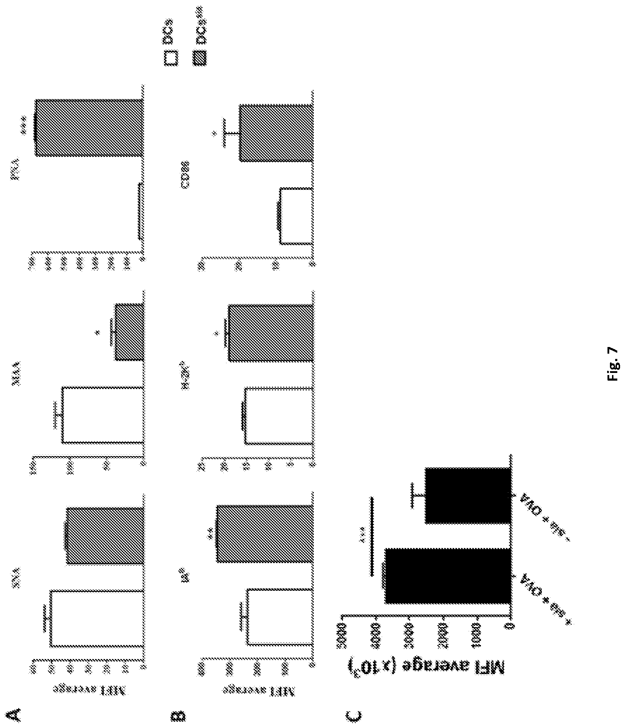

**Figure 7****.** Sialidase treatment improves maturation of murine DC and presentation of the ovalbumin model antigen. (A) Sialidase treatment of splenic DC removes α2,6- and α2,3-linked sialic acids. Murine splenic DC were treated with sialidase (DC^{sia}; black bars) or left untreated (DC; white bars), stained with *Sambucus nigra lectin* (SNA; recognizing α2,6-sialic acids), *Maackia amurensis lectin* (MAA; recognizing α2,3-sialic acids) and *Peanut agglutinin lectin* (PNA; recognizing unsialylated core 1 structures) and analysed by flow cytometry. Values represent the mean fluorescence intensity (MFI) ± SEM of at least six independent assays. (B) Evaluation of maturation and co-stimulatory markers after sialidase treatment. Murine DC were treated with sialidase (grey bars) or left untreated (white bars) and the maturation markers were evaluated by flow cytometry. Comparing untreated and sialidase treated DC, the MHC class II (IA^{B}) increased (15.3 ± 0.5 vs. 18.9 ± 0.7 MFI, *P< 0.05), as well as MHC class I (H-2K^{b}) (236.8± 1.6 vs. 345 ± 2.9 MFI, **P<0.001), as well as the co-stimulatory molecule CD86 (8.9 ± 0.6 vs. 19.7 ± 4.3 MFI, *P< 0.05). Graphs show the MFI ± SEM of six independent assays. (C) Sialidase treated DC show improved presentation of ovalbumin-derived peptide SIINFEKL, bound to MHC class I. Murine DC cells were first treated or not with sialidase and then incubated with ovalbumin. After these treatments the presentation of the ovalbumin-derived peptide SIINFEKL bound to H-2K^{b} (murine MHC class I) epitopes was analysed by flow cytometry, using the 25-D1.16 monoclonal antibody. Untreated DCs show an average MFI of 2525± 403.4, while sialidase treated DCs show an average MFI of 3692 ± 139.3. Thus the sialidase treatment improved 1.5-fold the reactivity of 25-D1.16 antibody, demonstrating higher presentation of the SIINFEKL peptide through MHC class I, which means a 1.5-fold increase in antigen cross presentation. Graphs show the MFI ± SEM of two independent assays. Statistical significance (***P < 0.0001) refers to the difference between untreated and sialidase treated DC and it was determined using a two-tailed paired t-test.

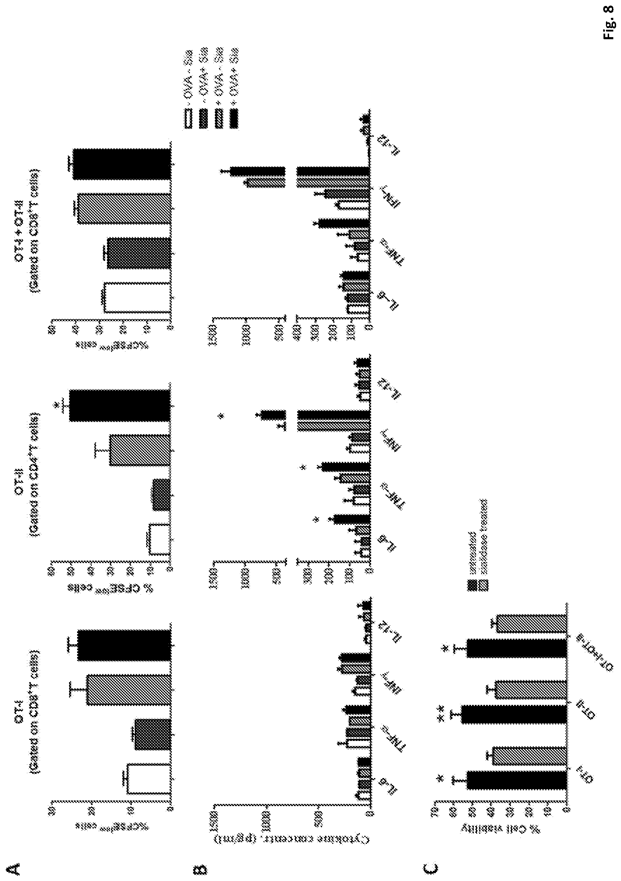

**Figure 8****.** Sialidase treatment of murine DC enhances their ability to activate ovalbumin specific T cells against ovalbumin expressing tumour cells. Murine splenic DC were pulsed with OVA for 4 hours and treated or not with sialidase for 1 hour. Splenocytes from OT-I or/and OT-II mice were isolated and co-cultured with DC for 72 hours in a ratio of 1:2 (DC: splenocytes). (A) After 72 hours, the percentage of proliferating ovalbumin-specific CD8⁺ (OT-I) or/and CD4⁺ (OT-II) T cells was determined by using the CFSE dilution assay. Flow cytometry analysis showed an increase in proliferation of OT-I T cells co-cultured with sialidase treated OVA-pulsed DC (+OVA+sia; black bars) (23.47 ± 2.439% of CFSE^{low} cells), compared with those co-cultured with untreated OVA-pulsed DC (+OVA-sia; light grey bars) (21.23 ± 4.288% of CFSE^{low} cells). As for the OT-II T cell sialidase treated OVA-loaded DC (+OVA+sia) significantly increase the proliferation of OVA-specific CD4⁺ T cell, compared with untreated OVA-loaded DC (+OVA-sia) (50.68 ± 3.50 vs 30.43 ± 7.33 % CFSE^{low} cells, *P< 0.05). Thus sialidase treatment of murine DCs improved their ability to induce the proliferation of: OT-I in 9%, and of OT-II in 66%. Graphs show the values of mean percentage ± SEM of CFSE^{low} cells. Statistical significance (*P < 0.05) refers to the difference between untreated and sialidase treated DC of at least two independent assays and was calculated using a two-tailed paired t-test. (B) Levels of IFN-y, IL-6, IL-12 and TNF-α in the supernatants of co-cultures were assessed by ELISA. Significant differences were found I the case of OT-II cells and not in the case of OT-I and OT-I plus OT-II. With OT-II, the TNF-α production was significantly up-regulated in the co-culture of OT-II splenocytes plus sialidase treated OVA-pulsed DC (+OVA+sia) compared to the untreated ones (+OVA-sia) (228.9 ± 22.36 vs 143.5 ± 25.48 pg/mL, *P< 0.05). The production of IL-6 (170.7 ± 26.46 vs 67.24 ± 33.64 pg/mL, *P< 0.05) and IFN-γ (734.0 ± 86.45 vs 370.6 ± 102.7 pg/mL, *P< 0.05) was also significantly upregulated. Thus sialidase treatment of murine DCs improves the ability of CD4 T cells to produce 1.6-fold more TNF-α, 2.60-fold more IL-6 and 1.98-fold more IFN-y. A tendency of a higher production of TNF-α and IFN-y secretion was observed after OT-I plus OT-II cells were co-cultured together with sialidase treated OVA-pulsed DC compared with untreated ones. Graphs show the mean ± SEM of cytokine concentration (pg/mL) for at least 3 independent assays. Statistical significance was determined using a two-tailed paired t-test. (C) Induction of cytolysis on tumour targets was investigated by culturing untreated (untreated; black bars) or sialidase treated (sialidase treated; grey bars) OVA-pulsed DC, splenocytes from either OT-I or OT-II mice or both and OVA-expressing B16 mouse melanoma cells (B16-OVA) for 24 hours. B16-OVA tumour cell death was assessed by staining with 7-ADD and annexin-V and analysed by flow cytometry. Sialidase treated OVA-pulsed DC induced a higher B16-OVA cell cytotoxicity compared to untreated ones. Graphs show the values of mean percentage ± SEM of viable cells of two independent assays. Statistical significance (*P < 0.05 or **P < 0.001) refers to the difference between untreated and sialidase treated DC and was calculated using a two-tailed paired t-test.

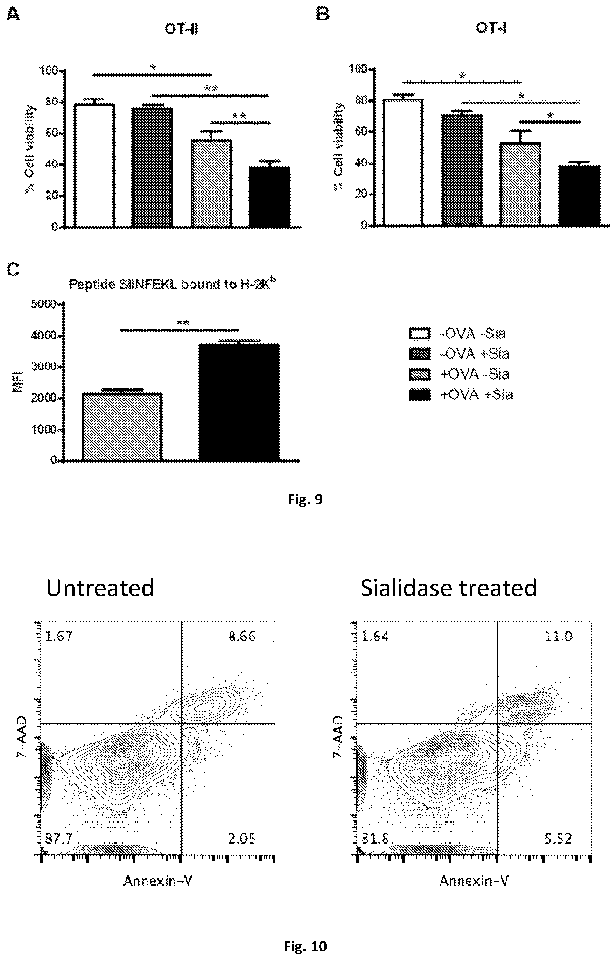

**Figure 9****.** Sialidase treatment of murine sDCs loaded with OVA improves T cell cytotoxicity against tumour cells. Induction of cytolysis of tumour targets was tested by co-culturing untreated (light grey bars) or sialidase treated (black bars) OVA-pulsed sDCs and splenocytes from either OT-II or OT-I mice for 72 hours. After, primed T cells and OVA-expressing B16 mouse melanoma cells (B16-OVA) were co-cultured for 24 hours. B16-OVA tumour cell death was assessed by staining with 7-AAD and annexin-V and analysed by flow cytometry. (A) OT-II cells that were pulsed with sialidase treated sDCs induced a higher tumour cell cytotoxicity compared to those pulsed with fully sialylated sDCs (55.68 ± 5.7 vs. 37.79 ± 4.5, 1.5-fold). (B) OT-I cells that were pulsed with sialidase treated sDCs induced a higher tumour cell cytotoxicity compared to those pulsed with fully sialylated sDCs (52.59 ± 7.9 vs. 37.86 ± 3.0, 1.4-fold). Statistical significance (*P < 0.05 or **P < 0.001) refers to the difference between fully sialylated and desialylated sDCs (n=2). (C) Sialidase treated sDCs show improved presentation of ovalbumin-derived peptide SIINFEKL, bound to MHC class I. Murine sDCs cells were first treated or not with sialidase and then incubated with ovalbumin. After these treatments the presentation of the ovalbumin-derived peptide SIINFEKL bound to H-2K^{b} (murine MHC class I) epitopes was analyzed by flow cytometry, using the 25-D1.16 monoclonal antibody. Sialidase treatment improved 1.7-fold the reactivity of 25-D1.16 antibody, demonstrating higher presentation of the SIINFEKL peptide through MHC class I. Graphs show the MFI ± SEM of two independent assays. Statistical significance (**P < 0.001) refers to the difference between untreated and sialidase treated DCs and it was determined using a two-tailed unpaired t-test.
**Figure 10****.** Sialidase treatment does not affect DC viability. Human MoDCs were treated with sialidase or left untreated and then submitted to a dual stained with Annexin V and 7-AAD and analysed by flow cytometry. Human MoDCs treated with sialidase exhibit no significant difference in cell viability, compared with untreated ones, suggesting that MoDCs can tolerate the sialidase treatment and remain viable to exert their immunologic function.

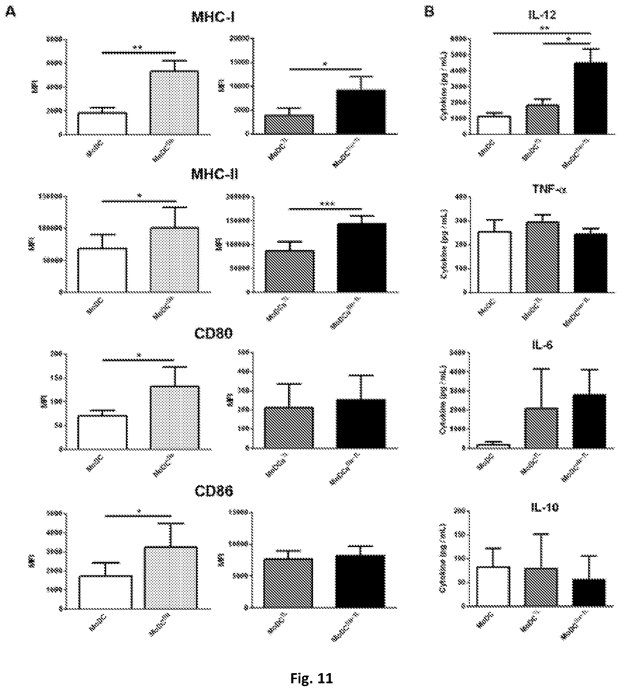

**Figure 11****.** Desialylated MoDCs show higher levels of maturation markers and expression of Th1 inducing cytokines. (A) Desialylated MoDCs show a higher maturation phenotype than fully sialylated MoDCs. The expression of several maturation markers was evaluated by flow cytometry. MoDCs were first treated with sialidase (Sia) for 1h at 37ºC and then loaded (A, right panel) or not (A, left panel) with lysates of the MCF-7 tumour cell line (TL), as a source of whole tumour cell antigens, as described in Example 5. Unloaded DCs that were treated with sialidase showed a significant increase expression of the antigen-presenting molecules MHC-I and MHC-II (2.9 and 1.5-fold, respectively), and of the co-stimulatory molecules CD80 and CD86 (1.9-fold in both cases). Similarly, a significantly improved maturation phenotype was observed after sialidase treatment of MoDCs loaded with lysates of the MCF-7 tumour cell line ; with a significant increase in the expression of MHC-I and MHC-II molecules (2.3 and 1.7-fold increase, respectively), and a slight increase of the co-stimulatory molecules CD80 and CD86 (1.2 and 1.1-fold increase, respectively). (B) Desialylated MoDCs, loaded with tumour antigens, show relevant alteration of cytokine secretion profile. IL-12, TNF-α, IL-6, and IL-10 cytokine secretion was evaluated by ELISA technique in culture supernatants obtained 24 hours after the treatments. The expression of IL-12 was 1823.00 ± 402.7 in MoDCs loaded with tumour antigens (MoDC^{TL}) and 4481.00 ± 869.9 pg/mL in MoDCs treated with sialidase and loaded with tumour antigens (MoDC^{Sia+TL}). Thus, the sialidase treatment increased 2.5-fold the expression of IL-12. The expression of IL-10 was 79.50 ± 44.02 pg/mL in MoDC^{TL} and 56.44 ± 49.44 pg/mL in MoDC^{Sia+TL}. Thus, sialidase treatment decreased 1.4-fold the IL-10 secretion. IL-6 and TNF-α expression was not significantly altered after sialidase treatment, showing that there is not an excessive pro-inflammatory cytokines release after sialidase treatment. This is recommendable in vaccination settings to avoid excessive pro-carcinogenic inflammation. Graph values represent the mean concentration (pg/mL) ± SEM of at least three independent assays. Statistically significant differences were calculated using a two-tailed paired t-test (*P < 0.05).

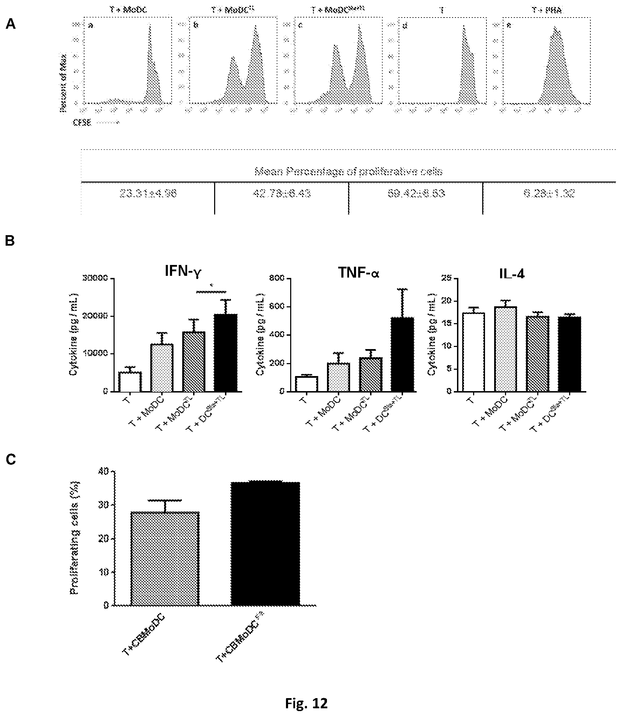

**Figure 12****.** Sialidase treatment of human adult or cord blood MoDCs improves their ability to activate T cells. MoDCs were first treated with sialidase (Sia), for 1 hour at 37ºC or left untreated, followed by loading with lysates of the tumour cell line MCF-7 (TL), as source of whole tumour cell antigens, as described in Example 5. MoDCs were then used in co-cultures with autologous T cells for 4-8 days in the presence of IL-2. (A) Induction of T cell proliferation by MoDCs. T cells were previously labeled with CFSE and the progeny, i.e., the percentage of T cells that proliferated was estimated by flow cytometry, based on the dilution of CFSE. The histograms show representative experiments with T cells that were co-cultured with: unloaded MoDCs (panel a), with MoDCs loaded with tumour cell line MCF-7 lysates, as source of whole cell antigen (panel b) or with sialidase treated MoDCs loaded with MCF-7 cell line lysates (panel c). Unstimulated T cells (panel d) served as a negative control and phytohaemagglutinin-stimulated T cells (panel e) as a positive control. Using MoDCs loaded with tumour cell lysates (MoDC^{TL}) resulted in 42.78 ± 6.43 % of proliferative T cells, a 6.8-fold increase when compared to T cells in culture alone (6.28 ± 1.32 %), and a 1.6-fold increase when compared to T cell in culture with MoDCs (23.31 ± 4.97 %). Moreover, treating MoDCs with sialidase and loading them with tumour cell lysates (MoDC^{Sia+TL}) led to a percentage of proliferative T cells of 59.42 ± 8.53 %, an increase of 9.5-fold when compared to T cells alone, 2.5-fold, when compared to T cells in culture with MoDCs and 1.4-fold compared to T cells in culture with MoDC^{TL}. Tabled values represent the mean percentage of proliferative cells ± SEM of 4 independent measurements. (B) Th1 cytokines secreted in MoDC-T cell co-culture. The cytokines secreted into the co-culture supernatants, following T cell stimulation with MoDCs were measured by ELISA. In the case of IFN-y, T cells boosted by Mo-DCs secreted 12489 ± 3067 pg/mL, which is 4.1-fold more than T cells alone. When stimulated by MoDC^{TL}, or MoDC^{Sia+TL} the secretion of IFN-γ increases to 15784± 3360 or 20392 ± 3966 pg/mL, respectively. Thus sialidase treatment prior to the upload of DCs with tumour antigen improves 1.3-fold their capacity to activate the secretion of IFN-γ by autologous T cells. T cell stimulated by MoDCs expressed 200.1 ± 72.5 pg/mL of TNF-α, which is 1.9-fold more than T cell alone. When boosted by MoDC^{TL}, or MoDC^{Sia+TL} the TNF-α secretion increases to 241.9 ± 56.9 and 514.6 ± 206.8 pg/mL, respectively. Thus sialidase treatment, prior to the upload of DC with tumour antigen improves 2.1-fold the ability of autologous T cells to secrete TNF-α. The expression of IL-4 by T cells was not altered when primed either by fully sialylated or desialylated MoDCs. Graph values represent the mean concentration (pg/mL) ± SEM of at least six independent experiments. Statistically significant differences were calculated using t-test (*P < 0.05). (C) Sialidase treatment of cord blood MoDCs (CBMoDCs) improves allogenic T cell proliferation. Cord blood MODCs were treated with sialidase (Sia), for 1 hour at 37ºC or left untreated, as described in Example 2. Stimulation of T cell proliferation was performed in a mixed lymphocyte reaction, where CBMoDCs were used in co-cultures with allogenic adult T cells for 7 days in the presence of IL-2. T cells were previously labelled with CFSE and the progeny, i.e., the percentage of T cells that proliferated was estimated by flow cytometry, based on the dilution of CFSE as described in Example 3. When T cells were stimulated by CBMoDCs, the percentage of proliferating cells was 27.75 ± 3.75; this value rose to 36.63 ± 0.49 (1.3-fold increase) when T cells were stimulated by CBMoDCs that have been previously submitted to sialidase treatment. Graphs show the mean ± SEM of % proliferating cells of two independent assays.

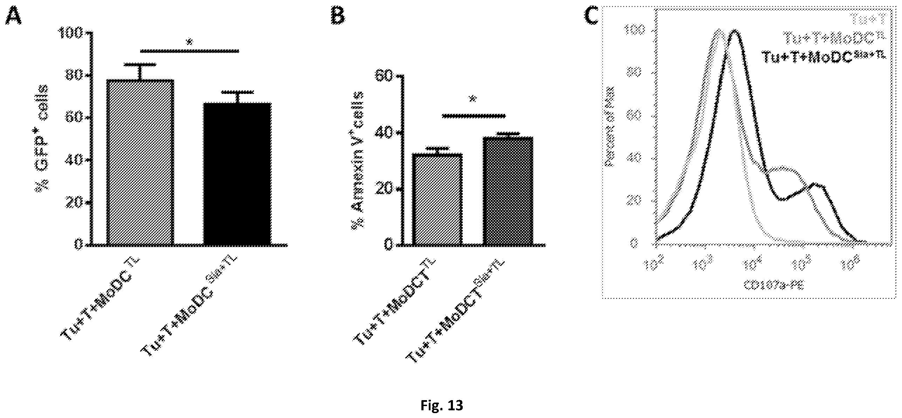

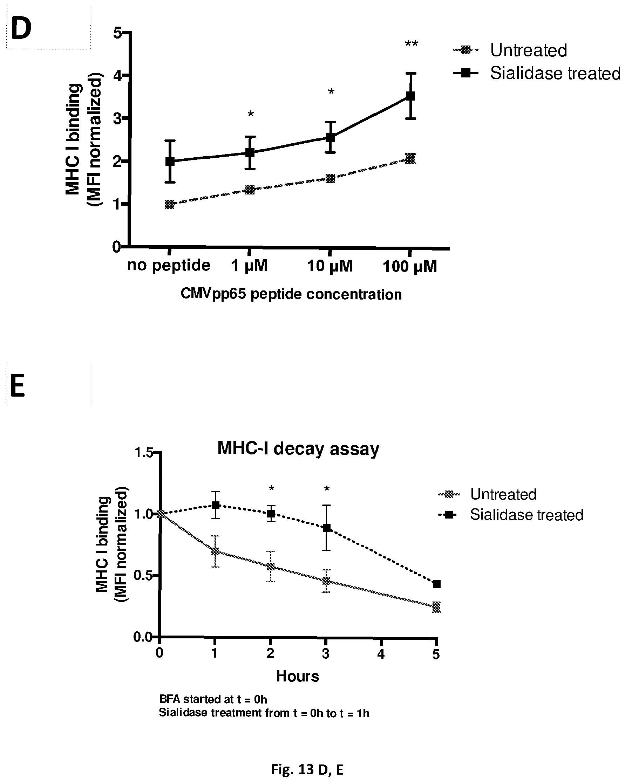

**Figure 13****.** Sialidase treatment of human MoDCs loaded with whole tumour cell antigens improves T cell cytotoxicity against tumour cells. MoDCs obtained from HLA-A*02:01 donors were treated with sialidase (Sia), for 1 hour at 37ºC or left untreated, followed by loading with lysates of the breast cancer cell line MCF-7 (TL), as a source of whole tumour cell antigens, as described in Example 5, thus obtaining MoDC^{Sia+TL} and MoDC^{TL}. Next, autologous CD3⁺ cells were co-cultured with those MoDCs for 3 weeks. CD3⁺ cells were then co-cultured with MCF-7-GFP cells in a ratio of 1 tumour cell: 10 T cells for 5 hours. (A) Tumour cell viability is decreased when T cells are challenged with desialylated DCs. Tumour cell viability was evaluated based on the expression of GFP in tumour cells. By measuring the expression of the GFP, in GFP-expressing tumour cells, it is possible to infer which cells are viable. GFP is a pH sensitive dye which fluorescence decreases significantly when the pH of the cell cytoplasm decreases, as it is the case of T cell-dependent apoptosis. Tumour cell viability was significantly decreased when incubated with T cells that had been previously primed with desialylated MoDCs loaded with tumour-antigens (MoDC^{Sia+TL}; 66.50 ± 5.3 %), compared to those incubated with T cells primed with fully sialylated MoDCs (MoDC^{TL}; 77.53 ± 7.8 %). Graph values represent the percentage ± SEM of viable tumour cells (GFP⁺) of four independent assays. (B) Tumour cell death is increased is decreased when T cells are challenged with desialylated DCs. Tumour cell loss of viability was evaluated based on the annexin V and 7-AAD staining. Non-stimulated T cells were cultured with MCF-7-GFP as control (data not shown). Graph represents the percentage ± SEM of apoptotic tumour cells assessed by staining with Annexin-V and 7-AAD, in 3 independent measurements. Tumour cell apoptose was significantly increased when cells were incubated with T cells that had been previously primed with desialylated MoDCs loaded with tumour-antigens (MoDC^{Sia+TL};37.95 ± 1.83 %), compared to those incubated with T cells primed with fully sialylated MoDCs (MoDC^{TL};32.03 ± 2.47 %). (C) T cells cytotoxic activity against tumour cells was also evaluated based on their degranulation activity (expression of the CD107a marker). A representative flow cytometry histogram of 4 independent measurements showing CD107a expression at the surface of T cells. Degranulation of T cells against tumour cells was evaluated by CD107a expression. The highest degranulation of T cells was observed when the cells were primed with desialylated MoDCs, as shown by the higher number of T cells expressing cell surface CD107a. Thus, T cells stimulated with DC that were sialidase treated prior to the upload with tumour antigens, show 1.2-fold better ability to kill tumour cells than those that were stimulated with DCs loaded with antigens only

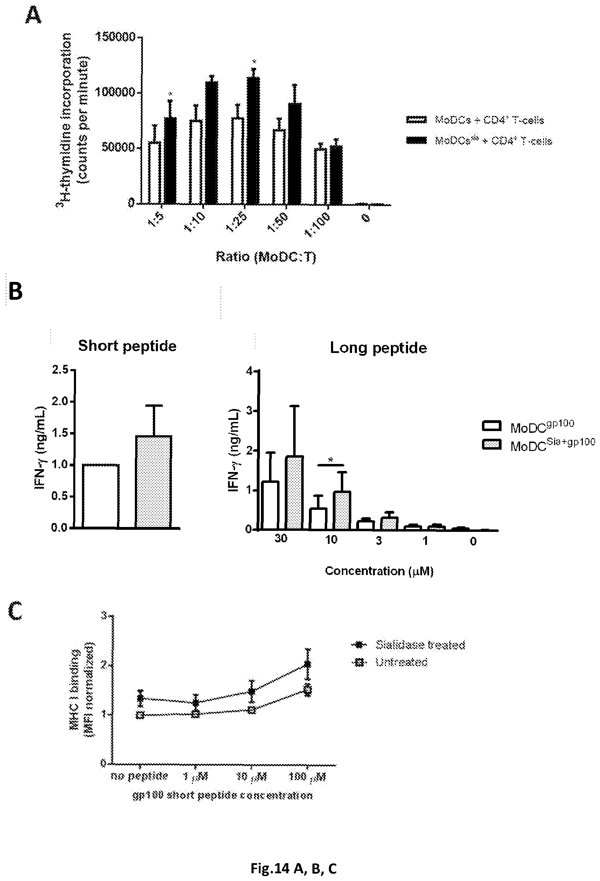

**Figure 14****.** Sialidase treatment improves the ability of MoDCs to activate tumour antigen gp100-specific T cells. (A) Improved proliferation of autologous helper CD4⁺ T cells, challenged for 3 days, with sialidase treated MoDCs. The proliferation of CD4⁺ T cells, challenged with different ratios of MoDCs treated (MoDCs^{sia}) or untreated with sialidase (MoDCs), was measured, by using the [³H]thymidine uptake assay (based on the scintillation counts of the cells). When comparing different MoDC:T cell ratios, the treatment of MoDCs with sialidase results in an increase of the scintillation counts per minute, relatively to the untreated condition. The lowest increase was 5% and occurred in the 1:100 ratio (49838.8 ± 5237.4 scintillations counts per minute using untreated MoDCs, vs. 52273.2 ± 6622.5 in the sialidase treated MoDCs). With the 1:50 ratio there was an increase of 36% (66786.0 ± 10693.6 vs. 90686.3 ± 17303.8 scintillations counts per minute (untreated vs. sialidase treated MoDCs)), the 1:5 ratio resulted in a 40% increase (55014.4 ± 15725.9 vs. 77147.2 ± 15801.5 scintillations counts per minute (untreated vs. sialidase treated MoDCs)) and with the 1:10 ratio there was a 46% increase (75085.2 ± 13916.8 vs. 109758.4 ± 5777.6 scintillations counts per minute (untreated vs. sialidase treated MoDCs)). Finally, the highest increase of the number of scintillations per minute occurred with the 1:25 ratio and corresponded to 48% (77250.3 ± 12477.3 *vs.* 114241.7 ± 8193.4 scintillations counts per minute (untreated vs. treated)) Graph values represent scintillation counts per minute ± SEM of three independent assays. The statistical significance, determined with two-tailed paired t-test (*P < 0.05), refers to the difference between untreated and sialidase treated MoDCs. (B) Improved antigen cross presentation by sialidase treated MoDCs. Gp100-specific CD8⁺ T cell were co-cultured overnight with gp100-loaded MoDCs, that have been previously treated or not with sialidase (MoDC^{sia+gp100} and MoDC^{gp100}, respectively). A short gp100 peptide, that has a high affinity and binds directly to the HLA-A*02:01, without previous endocytosis and processing was used to upload MODCs (left panel). Different concentrations of the long gp100 peptide, which is endocytosed and then cross-presented to CD8⁺ T cells, were used (right panel). The secretion of IFN-γ cytokine was measured by ELISA, to evaluate the activation of CD8⁺ T cells. The results were normalized to the IFN-y levels obtained when T cells were incubated with MoDCs loaded with the short peptide, without sialidase treatment and refer to the secretion ± SEM of IFN-y of three independent experiments. With the short peptide, using sialidase treated MoDCs, the expression of IFN-Y increased 49%, relatively to the condition with untreated MoDCs. When testing different long peptide concentrations, the results show that for the concentration of 30µM, there was a 64% increase for the sialidase treated MoDC condition. With a concentration of 10 µM, there was a significant 42% increase for the sialidase treated conditions. Using a concentration of 3µM of there was a 10% increase. Thus when using the long gp100 peptide as antigen, the sialidase treatment of the MoDCs resulted in an increase of IFN-Y secretion by gp100-specific CD8⁺ T cells. Therefore the production of IFN-y is improved up to 64% by gp-100 cytotoxic CD8⁺ T cells stimulated with sialidase treated MoDCs. (C) gp100 peptide bound MHC-I is more stable on sialidase treated T2 cells. T2 binding assays were performed by incubating cells, treated or not with sialidase, with different concentrations of gp100 short peptide in the presence of β-microglobulin for 3 to 4 hours at 37 °C. After incubation, T2 cells were washed and stained with anti-HLA-A*02 antibodies, and analysed by flow cytometry. Expression of HLA at cell surface was used as a measure to evaluate MHC stabilization. Data were normalized to the condition without peptide (no peptide) and without sialidase treatment. A higher stabilization of the HLA-A*02:01 molecule was observed after sialidase treatment, which ranged between 23% (1 µM of peptide) to 52% (100 µM of peptide). Using an intermediate peptide concentration (10 µM), the sialidase effect resulted in a stabilization effect of 38%. Graphs show the mean fluorescence intensity ± SEM of 4 independent experiments. (D) Accordingly, CMVpp65 peptide bound MHC-I is also more stable on sialidase treated T2 cells. T2 binding assays were performed as described before, using the CMVpp65 HLA-A*02:01 restricted peptide. Data were normalized to the condition without peptide (no peptide) and without sialidase treatment. A higher stabilization of the HLA-A*02:01 bound to CMVpp65 molecule was observed after sialidase treatment. A 87% increase in HLA-A*02:01 expression was observed when peptide concentration was 1 µM and a 146% increase was obtained for peptide concentration of 100 µM, when compared to condition without sialidase treatment. Using an intermediate peptide concentration (10 µM), the sialidase treatment resulted in a stabilization effect of 96% increase of HLA-A*02:01. Graphs show the mean fluorescence intensity ± SEM of 4 independent experiments. (E) Desialylation improves MHC-I stability on cell surface. T2 binding assays were performed by incubating cells with brefeldin A (BFA), blocking the Golgi network exporting nascent proteins to cell membrane. BFA was added and T2 cells were treated with sialidase as described before, in the presence of β-microglobulin. After 1 hour of treatment, sialidase was washed out but BFA was left in culture during the course of the experiment. At different time points, cells were stained with anti-HLA-A*02 antibodies, and analysed by flow cytometry. Data were normalized against the basal level of expression of HLA-A*02 by T2 cells. After 1 hour, T2 cells showed a 30% fold decrease in the expression of MHC-I. Conversely, sialidase treated cells were able to sustain MHC-I expression (8% increase). After 3 hours, sialidase treated cells still show a higher expression of HLA-A*02 molecules on cell surface (only 11% decreased) against 54% decrease in the condition without sialidase treatment. Graphs show the mean fluorescence intensity ± SEM of 6 independent experiments.

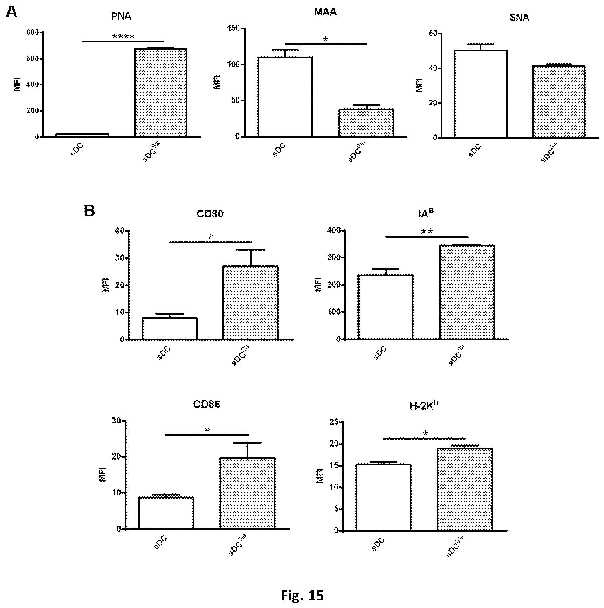

**Figure 15****.** Sialidase treatment improves maturation of murine DCs. (A) Sialidase treatment of splenic DCs removes α2,6- and α2,3-linked sialic acids. Murine splenic DC (sDCs) were treated with sialidase (sDC^{Sia}; grey bars) or left untreated (sDCs; white bars), stained with *Sambucus nigra lectin* (SNA; recognizing α2,6-sialic acids), *Maackia amurensis lectin* (MAA; recognizing α2,3-sialic acids) and *Peanut agglutinin lectin* (PNA; recognizing unsialylated core 1 structures) and analysed by flow cytometry. It was observed that sialidase treatment significantly decreased MAA binding (2.9-fold) and increased PNA staining (30.9-fold), resulting from the removal of α2,3-linked sialic acids. Removal of α2,6-linked sialic acids after sialidase treatment was also detected by SNA staining decrease (1.2-fold). Values represent the mean fluorescence intensity (MFI) ± SEM of at least six independent assays. (B) Evaluation of maturation and co-stimulatory markers after sialidase treatment. Murine DC were treated with sialidase (grey bars) or left untreated (white bars) and the maturation markers were evaluated by flow cytometry. Comparing untreated and sialidase treated DC, the MHC class I (H-2K^{b}) increased (15.3 ± 0.5 vs. 18.9 ± 0.7 MFI, 1.2-fold), as well as MHC class II (IA^{B}) (236.8 ± 1.6 vs. 345 ± 2.9 MFI, 1.5-fold), as well as the co-stimulatory molecules CD86 (8.9 ± 0.6 vs. 19.7 ± 4.3 MFI, 2.2-fold) and CD80 (7.8 ± 1.6 vs. 26.9 ± 6.1, 3.4-fold). Graphs show the MFI ± SEM of six independent assays. Statistical significance (*P < 0.05 and **P < 0.001) refers to the difference between untreated and sialidase treated DCs and it was determined using a two-tailed unpaired t-test.

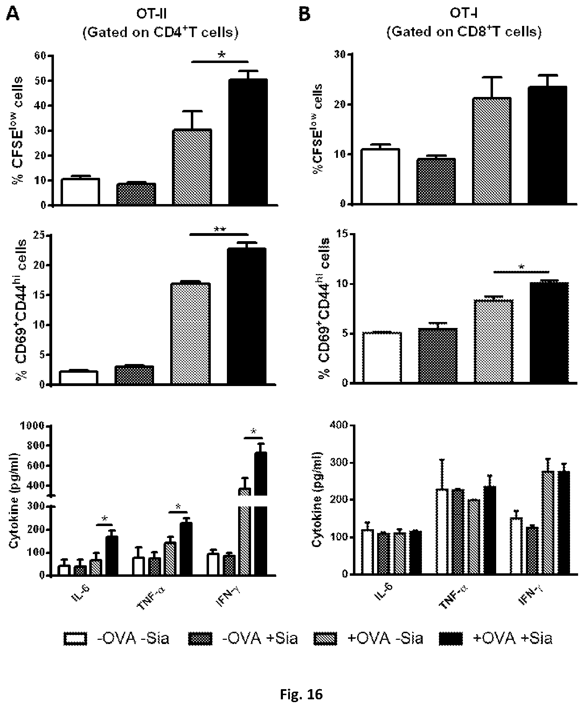

**Figure 16****.** Sialidase treatment of murine sDCs enhances their ability to induce proliferation and activation of ovalbumin-specific CD4⁺ and CD8⁺ T cells. Murine splenic DCs (sDCs) were pulsed with OVA for 4 hours and treated or not with sialidase for 1 hour. (A) Desialylated sDCs strongly induce proliferation, activation and differentiation of CD4⁺ T cells into a Th1 effector phenotype. Splenocytes from OT-II were isolated and co-cultured with the sDCs for 72h in a ratio of 1:2 (sDCs: splenocytes). After 72 h, the percentage of proliferating and activated CD4⁺ T cells was determined using a CFSE dilution and the expression of CD69 and CD44, respectively (gating on CD4⁺ T cells) was evaluated. Flow cytometry analysis show an increased proliferation of CD4⁺ T cells primed with fully sialylated OVA-pulsed sDCs, when compared with untreated ones (50.68 ± 3.50 vs 30.43 ± 7.33 % CFSE^{low} cells, 1.7-fold), as well an increased activation (22.77 ± 1.033% vs. 16.90 ± 0.4509, 1.3-fold). We assessed IFN-Y, IL-6, and TNF-α secretion in the supernatants of sDCs and T cell co-cultures by ELISA; a significant increase in all cytokines tested was observed. The concentration of TNF-α was significantly up-regulated in co-cultures of OT-II splenocytes with OVA-pulsed sDCs treated with sialidase compared to untreated ones (228.9 ± 22.36 vs. 143.5 ± 25.48 pg/mL, 1.6-fold), as well as the concentration of the pro-inflammatory IL-6 (170.7 ± 26.46 vs 67.24 ± 33.64 pg/mL, 2.6-fold) and the Th1 cytokine IFN-γ (734.0 ± 86.45 vs 370.6 ± 102.7 pg/mL, 2.0-fold). Statistical significance (*P < 0.05 or **P < 0.001) were calculated using unpaired t-test and refers to the difference between fully sialylated and desialylated sDCs. B) Desialyated sDCs induce activation of CD8⁺ T cells. Splenocytes from OT-I were isolated and co-cultured with sDCs for 72h in a ratio of 1:2 (sDC: splenocytes). The proliferation and activation of CD8⁺ T cells was determined by CFSE dilution and cell surface expression of CD69 and CD44 markers (gating on CD8⁺ T cell). Sialidase treatment of sDCs showed a tendency to induce a higher proliferation of CD8⁺ T cells compared to untreated sDCs (23.47 ± 2.439 vs. 21.23 ± 4.288% of CFSE^{low} cells). Furthermore, CD8⁺ T cells that were treated with sialidase showed a significant expression of CD69 and CD44 markers compared to fully sialylated OVA-pulsed sDCs (10.09 ± 0.2583 vs. 8.320 ± 0.4136%, 1.2-fold). The levels of IFN-y, IL-6, and TNF-α production in the supernatants of co-cultures were evaluated by ELISA. Graphs show the mean ± SEM of cytokine concentration (pg/mL) for at least 3 independent assays. Statistical significance was determined using a two-tailed unpaired t-test.

### Detailed description

The embodiments relating to T2 cells are presented for illustration purpose only.

The present disclosure relates to a method of obtaining a novel DC population with low sialic acid content, shows high MHC class I expression due to higher stability of the molecule at cell surface, high maturation and co-stimulatory properties, and less tolerogenic properties, suitable for cellular vaccine development. These DC are loaded with specific antigens, including peptides, proteins, or whole cell antigens, in particular tumour antigens, viral antigens and/or bacterial antigens, which are presented to T cells, and are capable of boosting antigen-specific T cell activity. These DCs are used to present several antigens to T cells, including tumour, viral or bacterial antigens, and are capable of boosting antigen-specific T cell activity. This disclosure also relates to the preparation method of such a mature DC population by cleaving sialic acids from living, viable human DC with sialidase, with the purpose to improve their immune potency.

The DCs generated by sialidase treatment show a decrease in the sialic acid expression, in particular a 50% decrease of the expression of α2,6 sialic acid, 45% decrease α2,3 sialic acid and to a lesser extent a decrease of the expression of α2,8 sialic acid. Functionally these DCs show:
i) improved expression of MHC class I and II molecules, in particular 2.3 and 1.7-fold increase, respectively;
ii) up to 146% of higher stability of MHC class I on cell surface and improved binding of exogenously supplied high affinity peptides that can bind directly to MHC-I, without previous endocytosis and processing;
iii) increased expression of co-stimulatory molecules, CD80 and CD86, in particular 1.2 and 1.1-fold increase, respectively;
iv) higher secretion of pro-inflammatory cytokines, such as IL-12 with 2.5-fold increase; and 1.4-fold decreased secretion of anti-inflammatory cytokines;
v) higher capacity to activate T cells with a Th1 phenotype, that produces 2.1-fold more TNF-α and 1.3-fold more IFN-γ and to expand 1.4-fold better the T cells;
vi) 20% higher capacity to induce T cell cytotoxic activity towards tumour cells;
vii) up to 1.7-fold higher antigen cross-presentation, guarantying a better activation of cytotoxic T cells.

The DCs are generated by sialidase treatment. Such treatment can be applied to DCs uploaded with target antigens, such as peptides, proteins, or whole cell antigens, which can be tumour antigens or other relevant antigens, such as viral and bacterial antigens.

The treatment of DCs with this single compound, the sialidase; provides all aspects of DC full maturation that are needed for effective cellular-mediated immune responses in DC-based vaccines.

In an embodiment, this method uses a single and defined enzyme, the sialidase, in contrast with the other protocols to prepare DC-based vaccines, which use cocktails of cytokines or bacterial antigens. The use of a single enzyme allows a cost-efficient method to activate DCs. The use of only one enzyme reduces excessive cell manipulation that leads to a significant decrease in DC viability and functionality.

The method of the present disclosure can be applied to different sources of DC, including DC derived from cord blood which are immature and tendentiously tolerogenic, due to maternal-fetal tolerance; DC differentiated from monocytes isolated from different blood sources, such as human adult peripheral blood and cord blood, or DC differentiated from CD34⁺ hematopoietic stem cells isolated from cord blood or other sources.

The method of the present disclosure, due to their very low toxicity on viable DC and high enzymatic activity, are useful for the *ex vivo* modification of glycans on the surface of DC.

The present disclosure relates to a new population of DCs and to an advantageous method for obtaining it. Such method consists in cleaving sialic acids from the surface of living and (viable) human DC, with the purpose of inducing maturation of antigen loaded DC-, including improving DC ability to present antigens as well as boosting antigen-specific T cell activity.

In an embodiment, the generation of DCs may be carried out as follows: DC can be isolated directly from blood or other tissue or obtained from differentiation of cell precursors, such as monocytes or CD34⁺ cells. DC generated from differentiation of blood precursors have the advantage to allow large amounts of DC.

In an embodiment, the DCs may be obtained from differentiation of monocytes, these are isolated from peripheral blood (PB) withdrawn from adult individuals or from cord blood (CB), and usually 0.5 to 1 × 10⁶ monocytes can be obtained per each ml of blood. The method to differentiate monocytes into DC is the method described by Sallusto & Lanzavecchia (1994), using IL-4 and GM-CSF cytokines, where the differentiation yields are above 70%, more particular above 90%, meaning that almost every monocyte will differentiate into DC. Peripheral blood can be, for example, obtained from leukapheresis from cancer patients or from blood donations, in case of healthy individuals, while CB can be obtained, for example, from a CB bank. The method of obtaining human DC can be as mentioned in Example 1, which includes DC obtained by differentiation of blood monocytes isolated from peripheral blood or from cord blood (Figures 1A and 1B, respectively).

In an embodiment, the sialidase treatment of human DC was carried out as follows: DC are submitted to an enzymatic treatment with an exogenous sialidase, that removes α2,3-, α2,6- and to a lesser extent α2,8- linked sialic acids.

In an embodiment, the sialidase can be recombinant or purified from biological sources. Sialidase can be from microbial, mammalian or other sources.

DC are desialylated using a sialidase at a concentration ranging frorr 0.025U/mL to 0.1U/mL, from 45 minutes to overnight, at 37ºC. The optimal proportion ranged between 0.002U/mL to 0.005U/mL per 1×10⁶ DCs. Sialidase treatment can be stopped by resuspending cells in complete media containing FBS or by diluting the sialidase reaction volume 10 times with culture medium.

In an embodiment, DCs may be desialylated using an optimal concentration of sialidase ranging from 0.002 U/mL to 0.005 U/mL per 1×10⁶ DCs at 37ºC and an optimal desialytation time of 45 minutes. These conditions led to the maintenance of a ~90 % DC cell viability, in particular 94.2±2.7% viability, in contrast with previous work were a different concentration of sialidase, in particular 2 U/ml of neuraminidase for 90 minutes was used.

In an embodiment, sialidase treated DC showed a 50 ± 26 % decrease in binding of *Sambucus nigra* lectin (SNA), a lectin that recognizes mainly sialic acid α2,6-linked to lactosamine (6' sialyl Galβ1,4-GlcNAc), and a 45 ± 10 % decrease in binding of *Maackia amurensis* lectin (MAA), a lectin that recognizes mainly sialic acid α2,3-linked to lactosamine (3' sialyl Galβ1,4-GlcNAc) or, linked to the Galβ1,3-GalNAc dissacharide (Figure 2A). SNA and MAA reduced binding proved that 50 % of the α2,6-sialic acid and 45% of the α2,3-sialic acid are efficiently removed from DC cell surface, by the sialidase treatment. By contrast, the reactivity of *Peanut agglutinin lectin* (PNA), a lectin specific for the non-sialylated O-linked chains (Galβ1,3-GalNAc), also known as T antigen, had a 70-fold increase after treatment (Figure 2A), thus confirming that a higher content of non-sialylated O-linked Galβ1,3-GalNAc structures is obtained at cell surface of DC after sialidase treatment. This reduction in 50% and 45% of α2,6- and α2,3-sialic acids, respectively, that is observed after sialidase treatment does not affect cell viability. Indeed, DCs maintain an average of 94.2±2.7% viability, after sialidase treatment, as assessed by staining with 7AAD (for dead cell exclusion) and annexin V (for apoptotic cell exclusion) (Figure10).

In an embodiment, the treatment with sialidase, according to the present disclosure, can be applied to human, murine and splenic DC, obtained as described in Examples 2 and 6, resulting in efficient reduction of sialic acid content at cell surface (Figures 7A and 15A).

In an embodiment, the antigen upload of sialidase treated DC was carried out as follows: DC to be used in vaccination protocols for cancer therapeutics, as well as immune or infection diseases, need to be uploaded with specific antigens.

In the example of tumour antigens, the term relates to any antigen expressed only by tumour cells or aberrantly expressed by tumour cells. It includes proteins that can be uptaken and processed by DC, being the derived peptides presented to cytotoxic and helper T cells via MHC class I or class II, respectively. To trigger an anti-tumour immune response in the host, either whole tumour cell antigens or specific antigens can be used. Examples of whole tumour cell antigens include tumour cell lysates, used in vaccination procedures; examples of specific tumour antigens used in vaccination comprise the gp100 glycoprotein, used as full length protein or as peptides specific for HLA-A*02:01.

In an embodiment, the sialidase treated DC can be uploaded with different types of antigens, as mentioned in the Examples 5, 6 and 7.

In an embodiment, for loading sialidase treated DC with whole tumour cell antigens, DC are incubated with the antigens in cell culture medium, for 3 to 4 hours, at 37ºC and 5% CO₂. Cell ratio can be the equivalent of antigens from 1 tumour cell or more per each 5 DCs. After incubation, antigen can be washed or maintained in culture. At this point, DC can be immediately co-cultured with the T cells or further activated with cytokines, such as TNF-α.

In an embodiment, the capacity of DC to phagocytose, i.e. to internalize the antigens, is preserved after sialidase treatment. No significant differences between sialidase treated and untreated DC, was observed regarding the endocytosis of whole tumour cell antigens (Figure 2B). Therefore, the present composition does not affect antigen phagocytosis. The fact that the endocytic capacity of DC was not affected after sialidase treatment was unexpected because sialidase treatment induces maturation of DC and it is generally accepted that DC when mature decrease their ability to endocytose. The fact that maturation is associated with decreased endocytosis ability explains why all the other existing protocols for DC loading with tumour antigen are performed before maturation stimuli. Inverting these procedures, i.e. performing maturation first and loading DC with antigens after is an advantage, that minimizes the problem of immune suppression, posed by tumour antigens when given to DC abrogate its maturation (i.e. impose a tolerogenic phenotype).

In an embodiment, sialidase treated DC showed an enhanced antigen presentation capacity and higher levels of co-stimulatory molecules.

In an embodiment, antigen loaded DC to be used in vaccination protocols need to show a mature phenotype which includes higher levels of MHC class I and class II antigen presenting molecules (signal 1) and the expression of co-stimulatory molecules, such as CD86 and CD40 (signal 2).

Surprisingly, sialidase treated DC that were loaded with antigen, show a higher maturation and co-stimulation profile (Figure 3and 11).

In an embodiment, as can be observed in Figure 3A and 11A, by using tumour cell lysates as an example of whole tumour cell antigen (Example 5), it is observed that when previously treated with sialidase according to Example 2, antigen loaded DC showed a marked higher expression of the antigen presenting molecules, MHC-I (9171±2910 vs 3949±1473; 2.3 fold increase) and MHC-II (MFI= 144106±16182 vs 86960±19666; 1.7-fold increase), and a slight increase of the co-stimulatory molecule CD80 (MFI= 251.8±129.5 vs 211.3±124.9; 1.2-fold increase) and CD86 (MFI= 8208±1447vs MFI = 7652±1341, 1.1-fold increase). Sialidase treatment of MoDCs that had not been loaded with tumour cell lysates showed a significantly increased expression of antigen presenting molecules MHC-I (MFI=5346±880.5 vs 1852±436.2; 2.9-fold increase) and MHC-II (MFI=101027±31935 vs 68504±22054; 1.5-fold increase) and of the co-stimulatory molecules CD80 (MFI=132.5±40.83 vs 70.78±11.02; 1.9-fold increase) CD86 (MFI=3242±1264 vs 1716±717; 1.9-fold increase).

In an embodiment, the combination of sialidase treatment with antigen upload leads to an increase in the expression of other co-stimulatory molecules such as CD40 (1.1 fold), CD80 (1.5 fold), CD83 (1.5 fold) and the expression of the chemokine receptor CCR7 (1.2 fold), when compared with untreated DC, based on the flow cytometric analysis of cells stained with appropriate antibodies. The expression of these co-stimulatory markers as well as the chemokine receptor guarantee that sialidase treatment generates DC with co-stimulatory activity and that are able to migrate to lymph nodes in response to chemokine gradients.

In an embodiment, the sialidase treated DC show enhanced expression of Th1-inducing cytokines.

In an embodiment, the functional maturation of DC is also evaluated by the type and amount of cytokines expressed by these cells.

In an embodiment, by using tumour cell lysates as an example of whole tumour cell antigen (Example 5), it is observed that when previously treated with sialidase according to Example 2, antigen loaded DC showed a significant higher secretion of IL-12 cytokine, (4914,83 ± 1039,63 preferably 4481 ± 869.9), when compared to non sialidase treated DC (2674 ± 46,25, preferably 1823 ± 402.7). Thus sialidase treatment increased 1.8 fold, preferably - 2.5 fold the expression of IL-12.

In an embodiment, the sialidase treated DC showed no significant increase in secretion of IL-6, and TNF-α showing that apart from IL-12, which has a pro-inflammatory and prominent role in the activation of anti-tumour response, there is not excessive pro-inflammatory cytokines release (Figure 3B and 11B).

In an embodiment, there is a decrease in the expression of the anti-inflammatory cytokine IL-10 from 79.50 ± 44.02 in MoDC loaded with tumour lysates (MoDC^{TL}) to 56.44 ± 49.44 in the condition where MoDCs were treated with sialidase (MoDC^{Sia+TL}). Thus, sialidase treatment resulted in a 1.4-fold decrease in IL-10 secretion.

In an embodiment, the sialidase effect was also observed in DCs generated from monocytes isolated from cord blood, which are tendentiously tolerogenic. These DCs were generated as described in Example 1, and treated with sialidase as described in Example 2. The effect of sialidase on the expression of antigen presenting molecule MHC-I and MHC II and co-stimulatory molecule CD86 was evaluated, by staining cells with appropriate antibodies, followed by flow cytometry analysis (as described in Example 1), 24 hours after the treatment.

Unexpectedly, the sialidase treatment of DCs derived from cord blood monocytes precursors significantly increased the expression of MHC-I (2.4 fold) and MHC-II (1.3 fold) molecules and the co-stimulatory molecule CD86 (2.0 fold) (Table I).

In an embodiment, the secretion of pro-inflammatory cytokines of the sialidase treated DCs derived from cord blood monocytes precursors was evaluated by ELISA, as described in Example 10. In the samples that showed secretion of IL-12, the sialidase treatment of the DCs increased 3.9-fold the IL-12 secretion, in comparison with untreated DCs. Similarly, within the samples that showed TNF-α secretion the sialidase treatment increased 1.5 fold the TNF-α secretion (Table I).

In an embodiment, the secretion of IL-6 and IL-10 was relatively low in cord blood monocyte-derived DCs and showed no significant alteration upon sialidase treatment. In spite of the pro-inflammatory profile of IL-6, low levels of IL-6 together with increased levels of other pro-inflammatory cytokines such as IL-12 are recommendable for vaccination settings, to avoid excessive pro-carcinogenic inflammation.

**Table I. Effect of sialidase treatment on the expression of antigen presentation (MHC-I e MHC-II) and co-stimulatory molecules (CD86) and the cytokines IL-12 and TNF-α by cord blood monocyte-derived DCs.**

| CONDITION | MHC-I* | MHC-II* | CD86* | IL-12 (pg/mL) | TNF-α (pg/mL) |
|---|---|---|---|---|---|
| Not treated | 2944.0 | 7836.0 | 362.0 | 137.2 | 147.5 |
| Sialidase treated | 7110.0 | 10266 | 731.7 | 530.0 | 227.9 |

| | | | | | |
|---|---|---|---|---|---|
| * Values represent the mean fluorescence intensity of the cells stained with appropriate fluorescence conjugated antibodies and analysed by flow cytometry. | | | | | |

In an embodiment, the sialidase treatment increases DC ability to activate T cells, in a whole tumour cell antigen model.

In an embodiment, the T cell activation is measured in different ways, including cell proliferation and cytokine expression. In this embodiment desialylated DC loaded with whole cell tumour antigens, provided by cancer cells lysates showed a 9.5-fold higher ability to induce proliferation of autologous T cells (59.42 ± 8.53 % of proliferated T cells); while, the presence of T cells stimulated by untreated MoDCs loaded with tumour cell lysates resulted in a 4.9-fold increase of proliferation capacity (42.78 ± 6.43 % proliferated T cells), as compared to unstimulated T cells. Thus, sialidase treatment led to a 1.4 -fold increase in the percentage of proliferative T cells (Figures 4A and 12A).

In an embodiment, analysis of cytokine expression showed that T cells activated by sialidase treated DC loaded with whole tumour cell antigens showed 2.1-fold increase in TNF-α levels and 1.3-1.4-fold increase, in particular a 1.4 fold increase, in IFN-y when compared to T cells in culture with MoDCs loaded with tumour lysates (Figures 4B and 12B). TNF-α and IFN-y are two Th1-inducing cytokines that synergize to induce anti-tumour immune responses. The expression of IL-10 and IL-4 is not induced showing that Th2-inducing cytokines are not stimulated.

In an embodiment, the MoDCs derived from cord blood, obtained as in Example 1, were tested for their capacity to activate T cells, after sialidase treatment. T cell proliferation was evaluated, in a Mixed Leukocyte Reaction, by CFSE dilution method as described in Example 3. The Cord Blood MoDC were treated or not sialidase and co-incubated with T cells. T cell proliferation was assessed after 7 days. As shown in Figure 12C, desialylated cord blood monocyte-derived DCs induced higher T cell proliferation.

In an embodiment, the antitumor activity mediated by T cells is improved by sialidase treated DC.

In an embodiment, the antitumor activity is measured in co-cultures using activated T cells against tumour cells. As described in Example 9, the cytotoxicity against tumour cells can be inferred by analysis of tumour cell viability or by measuring the release of CD107a (also called lysosomal-associated membrane protein-1 (LAMP-1)), which reflects the exocytosis of lytic granules from T cells.

In this embodiment desialylated DC loaded with whole cell tumour antigens were used to activate T cells, as in Example 3.

In an embodiment, the effector cells are CD3⁺ T cells that had been instructed by DC loaded with tumour lysates and treated or not with sialidase as in Example 2.

In an embodiment, the cytotoxicity of T cells against tumour cells was considered based on the loss of GFP fluorescence by tumour cells, when become apoptotic. Loss of tumour cell viability was also measured based on tumour cells reactivity with 7AAD and annexin V.

In an embodiment, the cytotoxicity against tumour cell was evaluated based on the release of cytotoxic granules from T cells, by evaluating CD107a expression.

In an embodiment and as shown in Figures 5B and 13A the viability of the tumour cell line MCF-7, that stably express GFP was evaluated. GFP is a dye whose fluorescence is pH sensitive, decreasing significantly when the pH of the cell cytoplasm decreases, as it is the case of cells that initiate apoptosis. The intensity of the fluorescence of the GFP, in GFP-expressing tumour cells, allows to infer which tumour cells survive or initiate apoptosis. Thus, by measuring the percentage of tumour cells still expressing GFP, hence alive, we have determined a spontaneous cell survival of 94%. As shown in Figures 5 Band 13A co-culturing tumour cells with T cells stimulated with MoDC treated with sialidase and loaded with tumour cell lysates, the percentage of surviving tumour cells decreases to 66.50 ± 5.73 % (i.e. reduce tumour cell viability up to 34%): this shows that sialidase treatment of MODCs leads to a better cytotoxic effect of T cells against the tumour cells than T cells previously stimulated with MoDC loaded with tumour cell lysates only, which showed a tumour cell survival of 77.53 ± 7.77 %. In Figure 13B, we evaluated tumour cell viability based on the annexin V and 7-AAD staining. Tumour cell apoptosis was significantly increased when cells were incubated with T cells that had been previously primed with desialylated MoDCs loaded with tumour-antigens (MoDC^{Sia+TL};37.95 ± 1.83 %), compared to those incubated with T cells primed with fully sialylated MoDCs (MoDC^{TL};32.03 ± 2.47 %).

In the representative histogram shown in Figure 13C, it is shown that when using T cells stimulated with MoDC that were treated with sialidase and loaded with tumour cell lysates, T cell degranulation increases compared to the condition where T cells were boosted with desialylated DCs loaded with tumour antigen.

In an embodiment, these two methods of evaluating cytotoxicity against tumour cells show similar results and led to conclude that T cell dependent tumour killing is 20% improved by activation with the desialylated DC composition.

In an embodiment, sialidase treatment improves DCs capacity to activate proliferation of helper T cell subpopulation.

In an embodiment, expansion of helper (CD4⁺) T cells is very important in the setting of antitumor immune responses. The role of CD4⁺ helper T cells in this response is largely attributed to providing regulatory signals required for the priming of antigen specific CD8⁺ cytotoxic T cells.

In this embodiment, desialylated human DC were able to expand autologous CD4⁺ helper T cells with better ability than untreated DC (Figures 6A and 14A). Sialidase improvement of CD4+ T cell proliferation varied between 5 and 48%, depending on the DC:T cell ratios. The best proliferation increases (average increase of 42.5%) were observed for the higher DC:T ratios from (1:5) to (1:50), while the effect was less pronounced, of only 5% for the lower ratio (1:100). Thus the present composition produces DCs with higher capacity to induce helper CD4⁺ T cells.

In an embodiment, the sialidase treatment improves DCs capacity to activate gp100 tumour antigen specific T cells and promotes cross-presentation.

In an embodiment, DCs were loaded with gp100 derived peptides according to Example 7: the gp100 long peptide (YLEPGPVTANRQLYPEWTEAQRLDC) is the SEQ ID 1 and the gp100 short peptide (YLEPGPVTA) is the SEQ ID 2.

In an embodiment, the aminoacid sequence that is shown underlined binds to HLA-A*0201, an allele of MHC class I. Both peptides are presented to CD8⁺, cytotoxic T cells. The long peptide has to be internalized, conducted into phagossomes and be processed before it undergoes cross-presentation. The short peptide has a high affinity and binds directly to MHC-I, in particular to HLA-A*02:01, at cell surface, without previous endocytosis and processing.

In this embodiment, activation of gp100 peptide-specific CD8⁺ cytotoxic T cells was evaluated by measuring IFN-y secretion.

In an embodiment, and as shown in Figure 14B (left panel), when using desialylated DC loaded with the gp100 short peptide to stimulate T CD8⁺ clones, the expression of IFN-y increased 49%, relatively to the condition with untreated MoDC.

Similarly, as shown in Figure 14B (right panel), the use of desialylated DCs, loaded with different concentrations of the gp100 long peptide to stimulate T CD8⁺ clones leaded to an increase in the secretion of IFN-γ in comparison with the parallel condition with DCs not treated with sialidase. The sialidase treatment of the MoDC resulted in an increase of IFN-γ secretion between 10% (with 3 µM), 42% (with 10 µM) and 64% (with 30 µM).

The data shows that sialidase treatment improves the capacity of DCs to activate gp100 tumour antigen specific T cells and promotes cross-presentation of antigens.

Cross-presentation is an essential mechanism for eliciting anti-tumour response because it allows DC to present external antigens that were phagocytosed, through MHC class I molecules, and thus activating antigen-specific cytotoxic T cells.

In an embodiment, the sialidase treatment improves MHC class I stability (Figures 14C, 14D e 14E).

In an embodiment, the efficient presentation of peptide-MHC class I complexes to CD8⁺, cytotoxic T cells benefits from a stable peptide-MHC-I interaction. In this embodiment, a T2 cell line that expresses unstable HLA-A*0201, was used to assess the effect of sialidase treatment in the stabilization of a gp100 and CMV pp65 peptides into the HLA-A*0201, as described in Example 8.

In an embodiment, the sialidase treatment of the T2 cell line resulted in an increased stability of the MHC-I HLA-A*0201 expression in the presence of the peptides (Figures 14C and 14D) demonstrating that sialidase treatment is able to improve between 23 and 52% peptide-MHC class I stability in the presence of gp100 peptide and from 121% to 256% in the presence of CMV pp65 peptide.

In an embodiment, the MHC class I stability was also 34% improved by sialidase treatment even in the absence of peptide, suggesting that sialidase treatment can improve MHC class I stability in a peptide independent manner (Figures 14C and 14D).

In an embodiment, to address whether the higher levels of MHC-I displayed at cell surface was due to higher synthesis of the molecule or due to a lower turnover rate that would increase MHC-I membrane half-life, we have used brefeldin A as described in Example 8. After 1 hour with BFA, T2 cells showed approximately 70% of the basal expression level of MHC-I (a decay of 30%). Conversely, sialidase treated cells were able to sustain MHC-I expression, displaying a slightly higher level (8%) of MHC-I molecules at cell surface. After 3 hours, sialidase treated cells still show a higher expression of HLA-A*02 molecules on cell surface, expressing approximately 90% (a decay of 10%)of the basal expression level of MHC-I. Non sialidase treated cells decreased the MHC-I expression to approximately 46% (a decay of 54%) after 3 hours in the presence of brefeldin A. These results show that stability of the MHC-I molecule at the cell surface is significantly increase (Figure 14E).

In an embodiment, the sialidase treatment improves DC maturation using ovalbumin as model antigen.

In an embodiment, to investigate the ability of the sialidase treatment to induce proliferation of antigen specific T cells, we have used ovalbumin as a model antigen and murine splenic DC (sDCs) (Example 6).

In an embodiment, the DCs treated with sialidase as in Example 2 and pulsed with ovalbumin (OVA) show reduced SNA and MAA lectin binding and increased PNA binding (Figures 7A and 15A), demonstrating the effectiveness of sialidase treatment.

In an embodiment, the sialidase treatment increased 23% the expression of MHC class I (from 15.3 ± 0.5 to 18.9 ± 0.7, MFI ± SEM ), 46% the expression of class II (from 236.8 ± 1.6 to 345 ± 2.9, MFI ± SEM ), as well as 121% the expression of the co-stimulatory molecule CD86 (from 8.9 ± 0.6 to 19.7 ± 4.3, MFI ± SEM) (Figure 15B), as compared to untreated sDCs.

In an embodiment, the sialidase treatment improves DC ability to activate T cells using ovalbumin as model antigen.

In an embodiment, the ability of murine DC, loaded with ovalbumin antigens, to activate helper or cytotoxic T cell responses is evaluated by using respectively transgenic OT-I (CD8⁺) or OT-II (CD4⁺) T cells, expressing T cell receptors (TCRs) specific for ovalbumin peptides, in the context of either MHC class I or class II.

In an embodiment, to assess the effects of sialidase treatment in this model system, sDC were loaded with OVA as in Example 6, and treated with sialidase as in Example 2 or left untreated and co-cultured with splenocytes isolated from OT-I or OT-II transgenic mice.

In this embodiment, the T cell activation was evaluated with a proliferation assay as in Example 3 and cytokine secretion as in Example 10.

In an embodiment, the proliferation of helper T cells (CD4⁺ T cells) was assessed using splenocytes from OT-II mice. Compared with untreated OVA-loaded sDCs (30.43 ± 7.33 of CFSE^{low} cells), sialidase treated OVA-loaded sDCs (50.68 ± 3.50 of CFSE^{low} cells) were found to significantly increase OVA-specific CD4⁺ T cell proliferation (6.9-fold increase) (Figures 8A and 16 A). Also, compared with untreated OVA-loaded DCs, sialidase treated OVA-loaded sDCs significantly increase activation of CD4⁺ T cells based on the increased percentage of CD69⁺ and CD44^{hi} phenotype (21.50 ± 1.450 vs 18.40 ± 1.411% of CD69⁺CD44^{hi} cells; Figures 8A and 16A).

In an embodiment, the levels of IFN-y, IL-6 and TNF-α in supernatants from the above-mentioned co-cultures were also evaluated by ELISA technique (Figure 8B and Figures 16A and 16B). The TNF-α production was up-regulated 60% in the co-culture of OT-II splenocytes plus sialidase treated OVA-pulsed DCs (228.9 ± 22.36 pg/ml) compared to the untreated ones (143.5 ± 25.48 pg/ml). Pro-inflammatory cytokines IL-6 and IFN-y production was also upregulated 154% (from 170.7 ± 26.46 to 67.24 ± 33.64 pg/ml) and 97% (from 730.0 ± 86.45 to 370.6 ± 102.7 pg/ml), respectively.

In an embodiment, the assessment of the proliferation of OVA-specific CD8⁺ T cells was performed. As shown in Figures 8A and 16B, OVA-loaded sDCs strongly induced OT-I proliferation, which was 11% potentiated by sialidase treatment of sDCs (23.47 ± 2.439 % of CFSE^{low} cells) when compared to untreated sDCs (21.23 ± 4.288 of CFSE^{low} cells). Also, compared with untreated OVA-loaded sDCs, sialidase treated OVA-loaded sDCs significantly increase activation of CD8⁺ T cells (10.09 ± 0.2583 vs 8.320 ± 0.4136%), based on the increased percentage of CD69⁺ and CD44^{hi} phenotype (Figures 8A and16B).

In an embodiment, the levels of IFN-y, IL-6 and TNF-α in supernatants from the OT-I co-cultures were also evaluated by ELISA technique (Figures 8B and 16B). However, no significant changes in cytokine production were detected among the conditions analysed, as it is observable on Figures 8B and 16B.

In an embodiment, the sialidase treatment improves DC ability to induce antigen-specific killing of tumour cells, using ovalbumin as model antigen.

In an embodiment, to investigate whether sialidase effect would translate into a higher ability of OVA-specific CD4⁺ and CD8⁺ T cells to induce tumour cytotoxicity, mouse melanoma cell line B16-OVA that stably expresses OVA (H2b) as described in Example 9 was used. As negative controls, splenocytes that were incubated with non-OVA pulsed DC were used.

In an embodiment, the B16-OVA tumour cells that were incubated with the negative controls showed a cell viability of more than 80% (not shown) compared to the ones that were stimulated with OVA-pulsed DC, for all conditions (Figure 8C and Figures 9A and 9B).

In an embodiment, the percentages of B16-OVA cell viability was significantly lower following exposure to splenocytes activated with sialidase treated OVA-pulsed DC, compared to the splenocytes activated with untreated OVA-pulsed DC. B16-OVA tumour cells that were incubated with either CD4⁺ (Figure 9A) or CD8⁺ T cells (Figure 9B), previously primed with sialidase treated OVA-pulsed sDCs, showed significantly less viability than those incubated with T cells primed with untreated OVA-pulsed sDCs (Figures 9A and 9B) (47% and 32% less, respectively).

In an embodiment, the sialidase treatment improves OVA peptide antigen specific presentation through MCH class I.

In an embodiment, the antigen specific presentation through MCH class I is essential to enable DC to activate antigen specific T cells. One of the tools to address this question consists in using murine DC loaded with the ovalbumin antigen.

In an embodiment, the presentation of the ovalbumin-derived peptide SIINFEKL through MHC class I (H-2Kb in mice) is easily accessed by flow cytometry using the 25-D1.16 monoclonal antibody.

In an embodiment, the DCs treated with sialidase and loaded with OVA showed a 1.5-fold increase in the MHC-I-dependent antigen presentation ability after sialidase treatment (Figure 9C).

### Examples

The following examples are provided merely as illustrative of various aspects of the present disclosure and shall not be construed to limit the disclosure in any way. The examples relating to T2 cells are presented for illustration purpose only.

### Example 1 - Generation and maturation of DC

By way of example, but not limited to, large amounts of DC can be obtained from differentiation of monocytes. DC isolated directly from blood or other tissue can also be used. Human monocytes can be isolated from peripheral blood (PB) withdrawn from adult individuals or from cord blood (CB). Peripheral blood can be, for example, obtained from blood donations, of healthy individuals, while CB can be obtained, for example, from a CB bank. PB and CB can be freshly processed or cryopreserved, for example, at -150°C. Added cryoprotectant agents can be 10% of dimethyl sulfoxide (DMSO), added drop-by-drop.

Cryopreserved cells can be unfrozen by, for example, a quick thawing (for approximately 1 minute) in a 37°C water bath, then suspending and mixing the cells with an equal volume of cell culture medium supplemented with 20% fetal bovine serum (FBS). After collecting cells by centrifugation, red blood cells can be lysed.

Before isolation of monocytes, mononuclear cells can be first isolated by, for example, density gradient centrifugations, using a Ficoll solution (Biochrom AG) in a 5:3 - 5:4 ratio, preferably a 5:3 ratio (blood dilution: Ficoll). Ficoll is a hydrophilic polysaccharide with 1.077 g/ml density, which is superior to PBMCs, but inferior to erythrocytes and granulocytes. Thus, during this centrifugation step, blood is separated by a density gradient, allowing the formation of four layers (from bottom to top of the tube): i) erythrocytes and granulocytes layer; ii) Ficoll solution layer; iii) a thin mononuclear cells layer; iv) and then a large layer, corresponding to plasma and the majority of the platelets. The mononuclear cell layer is washed using, for example phosphate buffer solution (PBS) and centrifuging cells twice, at 4ºC to improve removal of platelets.

Monocytes can be isolated by, for instance, positive selection using magnetic beads conjugated to antibodies against CD14, a marker mainly expressed by monocytes. For example, incubating mononuclear cells with CD14 Microbeads (Miltenyi Biotec), for 15 minutes, in the presence of beads buffer (PBS with 0.5% bovine serum albumin and 2 mM ethylenediamine tetraacetic acid (EDTA)). Cells are then loaded to a magnetic field of a MidiMACS Separator (Miltenyi Biotec) which will retain the cells labeled with the Microbeads (CD14⁺ cells or monocytes). Many commercially available media can be used to culture monocytes. By way of example, but not limited to, such media include: RPMI-1640 media with 10% FBS, 2 mM glutamax supplement with 100 µg/mL penicillin/streptomycin, 1% non-essential amino acids, 1% sodium pyruvate. To generate immature DC, the culture media for monocytes is supplemented with recombinant human (rh)IL-4 and rhGM-CSF for example, with 750 U/mL of IL-4 and 1000 U/mL of GM-CSF, and cultured at 37ºC, in a humidified atmosphere with 5% CO₂. The cells are cultured, for example, at 1×10⁶ cells/mL and half the media is replaced with fresh one every two days, during 5-7 days.

The generation of monocyte derived DC is confirmed by flow cytometry analysis, using markers for DC, for example CD1c or CD1a and the MHC class II molecules, the HLA-DR, as exemplified in Figure 1A and 1B.

Maturation of DC is crucial for the production of anti-tumour vaccines. For the maturation of DCs culture medium can be supplemented with inflammatory cytokines, for example, recombinant human TNF-α, IFN-y; and or bacterial components such as lipopolysaccharide, or viral double strand RNA such polyl:C and cultured at 37ºC, in a humidified atmosphere with 5% CO₂ for 24h to 48h.

The maturation of DC is confirmed by flow cytometry analysis, by evaluation of the expression of maturation markers for example MHC class II molecules, CD86, CD40, CD80 as exemplified in Figures 3A and 11A. Physiological maturation of DC is also evaluated by the expression of cytokines by ELISA as exemplified in Figures 3B and 11B.

### Example 2 - Sialidase treatment

The sialidase is used for its enzymatic activity against α2,3-, α2,6- or α2,8- linked sialic acids. In this example, DC are desialylated using a recombinant sialidase, at a concentration ranging from 0.025U/mL to 0.1 U/mL, from 45 minutes to overnight, at 37ºC. Control experiments are performed in parallel with heat-inactivated sialidase. The desialylation of DC, i.e. the efficiency of the sialidase treatment can be confirmed, for example, by staining with fluorescent lectins: fluorescein isothicyanate (FITC)-labeled Sambucus nigra lectin (SNA), Maackia amurensis lectin (MAA) and Peanut agglutinin lectin (PNA) and analysed by flow cytometry. SNA is a lectin which recognizes mainly sialic acid α2,6-linked to lactosamine (6' sialyl Galβ1,4-GlcNAc); MAA, is a lectin that recognizes mainly sialic acid α2,3-linked to lactosamine (3' sialyl Galβ1,4-GlcNAc) or, linked to Galβ1,3-GalNAc dissacharide, and PNA is a lectin specific for the non-sialylated O-linked chains (Galβ1,3-GalNAc, also known as T antigen). Thus, the degree of desialylation can be quantifiable based on the ratio of the mean fluorescence intensity (MFI) of DC after sialidase treatment compared to non-treated DC. Sialidase treatment leads to decreased SNA and MAA binding and increased PNA reactivity (Figure 2A). Sialidase treatment can be stopped by ressuspending cells in complete media containing FBS or by diluting the sialidase reaction volume 10 times with RPMI medium.

### Example 3 - Activation of T cells

T cells refer to a population of hematopoietic derived white blood cells, characterized by the expression of T cell receptors (TCRs), as well as the CD3 co-receptor. These cells also express CD4 and CD8 surface proteins associated with αβ T cell receptors, which characterize, respectively, helper and cytotoxic T cells. Helper T cells express several cytokines that are important for the activity of several other immune cells, including cytotoxic T cells. Cytotoxic T cells have the potential to kill cancer cells, cells that are infected (particularly with viruses), or cells that are damaged in other ways. Each T cell is specific for a given antigen presented via MHC by antigen presenting cells, such as DC.

By way of example, but not limited to, T cells can be obtained from PB, CB or other biological sources or alternatively, a human T cell line can be used. The T cell population can be, for example, the autologous CD14⁻ fraction obtained during monocyte isolation, which comprehends 75% of T cells. The T cell population or its subpopulations can be further enriched by, for example, positive selection using magnetic beads conjugated to antibodies, such as MicroBeads (Miltenyi Biotec), against CD3, CD4 or CD8. The T cells population or subpopulations can also be enriched by, for example, negative selection, using magnetic beads, conjugated to a cocktail of antibodies against every white blood cells except the desired population.

T cell proliferation can be measured for example by using the method of carboxyfluorescein succinimidly ester (CFSE) dilution, which relies on the dilution of a fluorescent dye from parental cells to daughter cells. T cells are resuspended at 50 × 10⁶/mL and labelled with 10 µM of CFSE solution, for 5 minutes at room temperature, in the dark. After washing, labelled cells are distributed in a 96 well U-bottom plate already containing or not the stimulator cells that can be mature DC. The ratio of DC:T can be 1:2 until 1:100. The T cells are incubated at 37ºC, and the CFSE intensity is evaluated at different times. rhIL-2 (10 lUg/mL) may be added at day 1, to help T cell division. The percentage of proliferative T cells is calculated based on the percentage of population that shows reduced CFSE expression by at least one cell division.

T proliferation can also be assessed by [³H]-thymidine incorporation into newly synthesized DNA molecules. After co-incubation of MoDCs with T cells for 3 days, [³H]-thymidine solution is added to the co-culture. After 24 h, cells are harvested into filters and [³H]-thymidine incorporation is assessed using scintillation counter that allow measuring the incorporation of radioactive thymidine into DNA of proliferating T cells.

### Example 4 - Cell viability

Cells are routinely counted, for example, by visualization at the optic microscope, and the viability can be assessed by trypan blue staining exclusion, using a Neubauer chamber. Trypan blue is used for example at 0.4% to discriminate dead cells (cells with blue coloration). Other cell viability exclusion stains can be used, such as the 7-Aminoactinomycin D (7AAD) (Sigma) or Live/dead fixable stain kit (Molecular Probes) that are used to identify dead cells, and annexin V that can be used to stain apoptotic cells, all of which are evaluable by flow cytometry. Moreover, tumour cell apoptosis may also be determined, based on the acidification of their cytoplasm. This acidification can be measured by using a reporter fluorescence, such as for example, in GFP-expressing tumour cells, whose GFP intensity decrease or is completely lost according to the acidification of the cell cytoplasm. Thus, when using tumour cell line expressing GFP, the determination of the percentage of cells that express or not GFP fluorescence after a certain treatment, may allow us to determine the percentage of cell survival.

### Example 5 - Testing efficacy of desialylated DC, using tumour cell lysates as a whole cell antigen model

In this example, the tumour antigens are from whole cells and obtained by lysing the cells (tumour cell lysates). Patient-derived cancer cells or available cell lines can be used to generate cell lysates, for example, the MCF-7 cell line, originally isolated from a pleural effusion of a 69-year-old Caucasian woman with a diagnosis of invasive breast ductal carcinoma (Soule *etal.,* 1973). MCF-7 cells express HLA-A*02:01, an HLAallele abundant in population. Therefore, this cell line functions as a target of T cells of HLA-A*02:01 individuals.

The cell lines can be labelled fluorescent, for example, the MCF-7 breast cancer cells stably expressing the green fluorescent protein (MCF-7-GFP) (Cell Biolabs). Many commercially available media can be used to culture cancer cell lines. By way of example, but not limitation, such media include: DMEM medium, supplemented with 10% FBS, 2mM L-Glutamine and 100 µg/mL penicillin/ streptomycin. Cells can be lysed, for example, through four freeze-thaw cycles, at -80ºC and 37ºC, respectively. Cell lysates are centrifuged to remove cell debris and the supernatants are stored at -80ºC. The cell lysis is confirmed, for example, at the optic microscope, by using trypan blue dye exclusion and by culturing an aliquot of lysate in complete culture medium during 2 days, to detect eventual cell growth. The concentration of protein in the tumour cell lysates is for example, 1 mg/mL.

For loading human DC with antigens derived from tumour cell lysates, DC can be, for example, incubated with antigens in complete RPMI medium, for 3 to 4 hours, at 37ºC and 5% CO₂. Cell ratio can be the equivalent of 1 or more tumour cells per each 5 DC. After incubation, antigen can be washed or maintained in culture. At this point, DCs can be immediately co-cultured with the T cells or further activated with cytokines, such as TNF-α.

### Example 6 - Testing efficacy of desialylated DC using ovalbumin (OVA) as a model antigen

In this embodiment, the ovalbumin (OVA) is used as a model antigen to load murine DC (sDCs) and to be presented to genetically modified T cells. T cells are from C57BL/6 transgenic mice, expressing OVA-specific, MHC class I and MHC class II restricted TCRs, named B6.OT-I and B6.OT-II (respectively). Mice can be 6-8 weeks old, age and sex matched and sacrificed by, for example, carbon dioxide inhalation followed by cervical dislocation.

Murine sDCs can be obtained by CD11c⁺ cell enrichment and incubated with OVA, for 3 to 6 hours at 37ºC in a humidified atmosphere with 5% CO₂.

Murine T cells are isolated for example, from animal spleens by mechanical disruption, followed by erythrocytes lysis. Splenocytes, from B6.OT-I and B6.OT-II mice, are resuspended, for example, at final concentration of 1 × 10⁷ /mL of media. T cell proliferation is estimated, for example, by the method of CFSE dilution as mentioned above.

### Example 7- Testing efficacy of using gp100 peptide as an antigen model

Tumour antigen for DCs loading can be a peptide derived from a tumour-associated protein. The peptide is screened for binding to specific MHC molecule, thus guarantying that it will be presented to peptide-specific T cells. In this example, the tumour antigen is a peptide derived from the gp100 protein, a known melanoma antigen. As example, two gp100 peptides can be used: gp100 short peptide (YLEPGPVTA) and gp100 long peptide (YLEPGPVTANRQLYPEWTEAQRLDC). The aminoacid sequence that is shown underlined binds to HLA-A*0201, an allele of MHC class I, frequent in population. The short peptide is internalized, directed into cytoplasm, transported into the endoplasmic reticulum (ER), and loaded on MHC class I molecules without being processed; then the peptide-MHC-I complex is further transported to the plasma membrane to be recognized by CD8⁺ cytotoxic T cells. The gp100 long peptide is internalized through phagocytosis and after processing it is cleaved. Then the resultant short peptide (underlined) becomes able to form a peptide-MHC-I complex that can be presented through MHC class I to CD8⁺ cytotoxic T cells, a phenomenon known as antigen cross-presentation. Therefore these epitopes also have the potential to be recognized by gp100 specific CD8⁺ T cell clones. For loading human DC with gp100 peptides, DC can be incubated with different concentrations of gp100 peptide, for example, 1 to 30 µM in RPMI medium, for 3 to 4 hours, at 37ºC and 5% CO₂. After incubation with antigens, DC are co-cultured with gp100-specific CD8⁺ T cell clones in IMDM medium. T cell clones activation can be evaluated, for example, by measuring the secretion of IFN-y cytokine by ELISA, after overnight or 3 days incubation.

### Example 8- MHC stability and MHC-peptide binding assay

In this example, T2 cell line (mutant LCL × T-lymphoblastoid hybrid cell line, 174 × CEM.T2) is used. T2 cells have a transporter associated with antigen transport (TAP) mutation that causes a peptide supply defect, i.e., they cannot incorporate peptides into MHC molecules. However, the T2 cells express stable MHC-I (HLA-A*02:01) molecules on their surface when an exogenous peptide is provided. Peptide binding assays can be performed by, for example, incubation of T2 cells, pre-treated or not with sialidase, with different concentrations of gp100 or CMVpp65 peptides in the presence of β-microglobulin for 3 to 4 hours, at 37 °C. After incubation, T2 cells are washed and stained with anti-HLA-A*02 antibodies and analysed by flow cytometry.

MHC-I cell surface stability can be assessed using the T2 cell line. T2 cells can be treated or not with sialidase in the presence of brefeldin A (BFA). Brefeldin A blocks protein transport from the endoplasmic reticulum to the Golgi network, therefore suppressing the incorporation of newly synthesized proteins into the cell membrane. In this context, T2 cells were treated or not treated with sialidase in the presence of BFA. After treatment, sialidase was washed out but BFA remained in the culture medium. At different time points up to 4 hours after treatment, T2 cells are washed and stained with anti-HLA-A*02 antibodies and analysed by flow cytometry.

### Example 9 - Cytotoxicity Assays

In this example, the cytotoxic capacity of T cells is evaluated using T cells boosted with autologous DC that have been subjected to different treatments. The cell ratio can be, for example, 1 DC per 5 T cells, and the incubation time of 7 to 21 days, with periodic reloads with DC submitted to specific treatments. T cells can be activated by addition of cytokines, for example, rhlL-7 (5 ng/mL). The proliferation of T cells can be stimulated by supplementation with rIL-2 (10UI/mL). Activated T cells are then cultured against target tumour cells that can be patient-derived cancer cells or commercially available cell lines. For example, the MCF-7-GFP cell line, mentioned above, can be pre-cultured in 96 well flat-bottom plates, for at least 4 h, at 37ºC. The cell ratio can be, for example, 10 or less T cells per each tumour cell. Co-cultures take place for 5 to 24 hours. The cell killing is evaluated by different strategies. In one example, tumour cell viability is estimated as mentioned in Example 4.

In other example, it is also possible to assess T cell cytolytic capacity by identifying the T cells that were specifically activated by the tumour cells. This can be achieved, for example, by staining molecules present in the membranes of lysosomes, such as CD107 molecules, that become exposed when T cells degranulate. Briefly, tumour and T cells can be co-cultured in the presence of the anti-CD107a antibody during 1 hour, at 37ºC. Then brefeldin A is added to the co-culture to block protein secretion, followed by incubation for 4 hours at 37ºC. After this period, the expression of the CD107a molecules can be evaluated by flow cytometry.

For experiments with murine cells, the mouse melanoma cell line B16-OVA was used as target cells and stained with CFSE, as described in Example3. Subsequently, the CFSE-labelled cells were seeded into 96-well microplates at a concentration of 0.1 × 10⁶ cell/well and incubated at 37ºC in 5% CO₂ incubator for 4 hours. The splenocytes from OT-I and OT-II mice were added to the microplates at a ratio of 5:1 (splenocytes: tumour cells) and cultured for 24 hours at 37ºC and 5% CO₂. Splenocytes from OT-I and OT-II mice were activated previously by incubation for 72 hours with OVA pulsed and non-pulsed mouse CD11c⁺ cells treated with sialidase or left untreated. After 24 hours, tumour cell killing is evaluated, as mentioned in Example 4.

### Example 10- Cytokine quantification

For the quantification of cytokine expression, culture supernatants were collected before cell harvest and frozen at -20°C. Concentrations of IL-4, IL-6, IL-10, IFN-y, TNF-α, IL-12, were quantified using ELISA technology as recommended by the manufacturer. Briefly, 96-well plates were coated with capture antibodies, overnight at 4ºC and then blocked for 1 hour at room temperature with 1% BSA. Next, the supernatants and standard concentrations of cytokines were incubated in duplicates or triplicates for 2 hours, at room temperature and washed 4 times with PBS-0.05%tween. Specific biotinylated detection antibodies were then added and incubated for 2 hours at room temperature, followed by 4 washes and horseradish peroxidase-conjugated-streptavidin incubation for 20 minutes. After 4 washes, plates were incubated with tetramethylbenzidine substrate in the dark and the reaction stopped by addition of 1M HCL. Bound complexes were then detected by A450 measurement on a plate reader and cytokine concentration was calculated as pg/ml using the standard curves.

### Example 11-Calculation of fold increase and fold decrease

For clarifying the terms used to present results, fold increase represents the ratio between value obtained for sialidase treatment condition and the corresponding value without sialidase treatment. Fold decrease represents the ratio between the value without sialidase treatment and the value obtained for sialidase treatment condition. For example, a 2 fold increase represents a 100% increase, and a 2 fold decrease represents a 50% decrease.

In an embodiment, a fold change is a measure of how much a quantity changes going from an initial value to a final value in comparison to a control value. Therefore, a fold change is a ratio.

Where singular forms of elements or features are used in the specification of the claims, the plural form is also included, and vice versa, if not specifically excluded. For example, the term "a cell" or "the cell" also includes the plural forms "cells" or "the cells," and vice versa. In the claims articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The disclosure includes embodiments in which exactly one member of the group is present in, employed in, or otherwise relevant to a given product or process. The disclosure also includes embodiments in which more than one, or all of the group members are present in, employed in, or otherwise relevant to a given product or process.

The following claims further set out particular embodiments of the disclosure.

### References:

[1] O. Acuto, S. Mise-Omata, G. Mangino, and F. Michel (2003). Molecular modifiers of T cell antigen receptor triggering threshold: the mechanism of CD28 costimulatory receptor. Immunol Rev 192, 21-31.
[2] J. Banchereau, and R.M. Steinman (1998). Dendritic cells and the control of immunity. Nature 392, 245-252.
[3] A. Buschiazzo, and P.M. Alzari (2008). Structural insights into sialic acid enzymology. Current opinion in chemical biology 12, 565-572.
[4] M.G. Cabral, Z. Silva, D. Ligeiro, E. Seixas, H. Crespo, M.A. Carrascal, M. Silva, A.R. Piteira, P. Paixão, J.T. Lau, and P.A. Videira (2013). The phagocytic capacity and immunological potency of human dendritic cells is improved by α2,6-sialic acid deficiency. Immunology 138, 235-45.
[5] H.J. Crespo, M.G. Cabral, A.V. Teixeira, J.T.Y. Lau, H. Trindade, and P.A. Videira (2009). Effect of sialic acid loss on dendritic cell maturation. Immunology 128, e621-e631.
[6] F. Dall'Olio, and M. Chiricolo (2001). Sialyltransferases in cancer. Glycoconj J 18, 841-50.
[7] M. Guadalupe Cabral, A. Rita Piteira, Z. Silva, D. Ligeiro, R. Brossmer, and P.A. Videira (2010). Human dendritic cells contain cell surface sialyltransferase activity. Immunology Letters 131, 89-96.
[8] A. Harduin-Lepers, V. Vallejo-Ruiz, M.A. Krzewinski-Recchi, B. Samyn-Petit, S. Julien, and P. Delannoy (2001). The human sialyltransferase family. Biochimie 83, 727-737.
[9] H. Jahnisch, S. Fussel, A. Kiessling, R. Wehner, S. Zastrow, M. Bachmann, E.P. Rieber, M.P. Wirth, and M. Schmitz (2010). Dendritic cell-based immunotherapy for prostate cancer. Clinical & developmental immunology 2010, 517493.
[10] H. Jonuleit, U. Kühn, G. Müller, K. Steinbrink, L. Paragnik, E. Schmitt, J. Knop, and A.H. Enk (1997). Pro-inflammatory cytokines and prostaglandins induce maturation of potent immunostimulatory dendritic cells under fetal calf serum-free conditions. Eur J Immunol 27, 3135-42.
[11] F. Lehmann, E. Tiralongo, and J. Tiralongo (2006). Sialic acid-specific lectins: occurrence, specificity and function. Cellular and molecular life sciences : CMLS 63, 1331-1354.
[12] R.L. Sabado, and N. Bhardwaj (2010). Directing dendritic cell immunotherapy towards successful cancer treatment. Immunotherapy 2, 37-56.
[13] F. Sallusto, and A. Lanzavecchia (1994). Efficient presentation of soluble antigen by cultured human dendritic cells is maintained by granulocyte/macrophage colony-stimulating factor plus interleukin 4 and downregulated by tumor necrosis factor alpha. J Exp Med 179, 1109-18.
[14] R. Schauer (2009). Sialic acids as regulators of molecular and cellular interactions. Current opinion in structural biology 19, 507-514.
[15] H.D. Soule, J. Vazguez, A. Long, S. Albert, and M. Brennan (1973). A human cell line from a pleural effusion derived from a breast carcinoma. J Natl Cancer Inst 51, 1409-16.
[16] R.M. Steinman (1996). Dendritic cells and immune-based therapies. Exp Hematol 24, 859-62.
[17] R.M. Steinman, and J. Banchereau (2007). Taking dendritic cells into medicine. Nature 449, 419-26.
[18] R.M. Steinman, and Z.A. Cohn (1973). IDENTIFICATION OF A NOVEL CELL TYPE IN PERIPHERAL LYMPHOID ORGANS OF MICE : I. MORPHOLOGY, QUANTITATION, TISSUE DISTRIBUTION. J Exp Med 137, 1142-62.
[19] R.M. Steinman, and Z.A. Cohn (1974). IDENTIFICATION OF A NOVEL CELL TYPE IN PERIPHERAL LYMPHOID ORGANS OF MICE : II. FUNCTIONAL PROPERTIES IN VITRO. J Exp Med 139, 380-97.
[20] R.M. Steinman, D.S. Lustig, and Z.A. Cohn (1974). IDENTIFICATION OF A NOVEL CELL TYPE IN PERIPHERAL LYMPHOID ORGANS OF MICE: III. FUNCTIONAL PROPERTIES IN VIVO. J Exp Med 139, 1431-45.
[21] A. Varki (2008). Sialic acids in human health and disease. Trends in molecular medicine 14, 351-360.
[22] A. Varki, R. Cummings, J. Esko, H. Freeze, P. Stanley, C. Bertozzi, G. Hart, and M. Etzler (2009). Essentials of Glycobiology.
[23] A. Varki, and P. Gagneux (2012). Multifarious roles of sialic acids in immunity. Ann N Y Acad Sci 1253, 16-36.
[24] N.M. Varki, and A. Varki (2007). Diversity in cell surface sialic acid presentations: implications for biology and disease. Lab Invest 87, 851-7.
[25] P.A. Videira, I.F. Amado, H.J. Crespo, M.C. Alguero, F. Dall'Olio, M.G. Cabral, and H. Trindade (2008). Surface alpha 2-3- and alpha 2-6-sialylation of human monocytes and derived dendritic cells and its influence on endocytosis. Glycoconjugate Journal 25, 259-268.

## Claims

1. A method for production of a viable cell population as comprising the steps of:
culturing isolated or obtained dendritic cell population or;
maturing the dendritic cell population by adding sialidase to dendritic cell population;
removing of α2,3-sialic acid, α2,6-sialic acid and α2,8-sialic acid from a cell surface of the dendritic cell population
wherein the dendritic cells are desialylated using a recombinant sialidase, at a concentration ranging from 0.025U/mL to 0.1 U/mL, from 45 minutes to overnight, at 37 °C.

2. The method according to the previous claim, wherein the removing step of α2,3-sialic acid, α2,6-sialic acid and α2,8-sialic acid from a cell surface precedes the maturing step.

3. The method according to claims 1-2 comprising a loading step of an antigen at the surface of the population of viable dendritic cells.

4. The method according to the previous claim 3 wherein the loading step of the antigen is performed at 37 °C, up to 4 hours, and up to 100 µM of said antigen.

5. The method according to claims 1-2 wherein the dendritic cell population is isolated from blood, from differentiation of monocytes, from CD34⁺ hematopoietic stem cells, or from the peripheral blood.

## Patentansprüche

1. Verfahren zur Herstellung einer lebensfähigen Zellpopulation, das die folgenden Schritte umfasst:
Kultivierung isolierter oder gewonnener dendritischer Zellpopulationen oder; Reifung der dendritischen Zellpopulation durch Zugabe von Sialidase zur dendritischen Zellpopulation;
Entfernung von α2,3-Sialinsäure, α2,6-Sialinsäure und α2,8-Sialinsäure von einer Zelloberfläche der dendritischen Zellpopulation
wobei die dendritischen Zellen unter Verwendung einer rekombinanten Sialidase bei einer Konzentration im Bereich von 0,025 U/ml bis 0,1 U/ml von 45 Minuten bis über Nacht bei 37 °C desialyliert werden.

2. Verfahren nach dem vorhergehenden Anspruch, wobei der Schritt der Entfernung von α2,3-Sialsäure, α2,6-Sialsäure und α2,8-Sialsäure von einer Zelloberfläche dem Reifungsschritt vorausgeht.

3. Verfahren nach Anspruch 1-2, umfassend einen Beladungsschritt mit einem Antigen auf der Oberfläche der Population lebensfähiger dendritischer Zellen.

4. Verfahren nach dem vorhergehenden Anspruch 3, wobei der Beladungsschritt mit dem Antigen bei 37 °C, bis zu 4 Stunden und bis zu 100 µM des Antigens durchgeführt wird.

5. Verfahren nach Anspruch 1-2, wobei die dendritische Zellpopulation aus Blut, aus der Differenzierung von Monozyten, aus CD34⁺ hämatopoetischen Stammzellen oder aus dem peripheren Blut isoliert wird.

## Revendications

1. Procédé de production d'une population cellulaire viable comprenant les étapes suivantes :
mise en culture de la population de cellules dendritiques isolée ou obtenue, ou ;
maturation de la population de cellules dendritiques en ajoutant de la sialidase à la population de cellules dendritiques ;
élimination de l'acide α2,3-sialique, de l'acide α2,6-sialique et de l'acide α2,8-sialique de la surface cellulaire de la population de cellules dendritiques où les cellules dendritiques sont désialylées à l'aide d'une sialidase recombinante, à une concentration allant de 0,025 U/ml à 0,1 U/ml, pendant entre 45 minutes et une nuit, à 37 °C.

2. Procédé selon la revendication précédente, dans lequel l'étape d'élimination de l'acide α2,3-sialique, de l'acide α2,6-sialique et de l'acide α2,8-sialique de la surface d'une cellule précède l'étape de maturation.

3. Procédé selon les revendications 1-2 comprenant une étape de chargement d'un antigène à la surface de la population de cellules dendritiques viables.

4. Procédé selon la revendication 3 précédente, dans lequel l'étape de chargement de l'antigène est effectuée à 37 °C, pendant jusqu'à 4 heures, et avec jusqu'à 100 µM de cet antigène.

5. Procédé selon les revendications 1-2, dans lequel la population de cellules dendritiques est isolée du sang, de la différenciation des monocytes, des cellules souches hématopoïétiques CD34+, ou du sang périphérique.
